(19) Europäisches Patentamt
European Patent Office
Office européen des brevets

(11) EP 0 566 459 B1

(12) **FASCICULE DE BREVET EUROPEEN**

(45) Date de publication et mention de la délivrance du brevet:
**03.03.1999 Bulletin 1999/09**

(21) Numéro de dépôt: **93400932.5**

(22) Date de dépôt: **09.04.1993**

(51) Int. Cl.$^{6}$: **C07B 41/02**, C07B 41/08, C07B 43/04, C07C 29/09, C07C 51/09, C07C 209/62, C07C 227/20, B01J 31/28

(54) **Réactif et procédé catalytique utile pour cliver une fonction protégée**

Reagenz und katalytisches Verfahren zur Spaltung von geschützten funktionellen Gruppen

Reagent and catalytic process for cleaving protected functional groups

(84) Etats contractants désignés:
**AT BE CH DE DK ES FR GB GR IE IT LI LU NL PT SE**

(30) Priorité: **15.04.1992 FR 9204621**

(43) Date de publication de la demande:
**20.10.1993 Bulletin 1993/42**

(73) Titulaire: **RHODIA CHIMIE**
**92408 Courbevoie Cédex (FR)**

(72) Inventeurs:
- **Blart, Errol**
  **F-94700 Maison Alfort (FR)**
- **Genet, Jean-Pierre**
  **F-91370 Verrieres le Buisson (FR)**
- **Savignac, Monique**
  **F-91190 Gif (FR)**
- **Bernard, Jean-Marie,**
  **Route du Large**
  **F-69440 Mornant (FR)**

(74) Mandataire:
**Ricalens, François et al**
**RHODIA CHIMIE**
**Direction de la Propriéte Industrielle**
**25, quai Paul Doumer**
**92408 Courbevoie Cédex (FR)**

(56) Documents cités:
**EP-A- 0 196 454**
**EP-A- 0 407 256**
**GB-A- 2 176 478**

- **JOURNAL OF ORGANIC CHEMISTRY. vol. 52, no. 22, 1987, EASTON US pages 4984 - 4993 O. DANGLES ET AL 'SELECTIVE CLEAVAGE OF THE ALLYL AND ALLYLOXYCARBONYL GROUPS THROUGH PALLADIUM-CATALYZED HYDROSTANNOLYSIS WITH TRIBUTYLTIN HYDRIDE. APPLICATION TO THE SELECTIVE PROTECTION-DEPROTECTION OF AMINO ACID DERIVATIVES AND IN PEPTIDE SYNTHESIS'**
- **Larousse Trois Volume en couleurs**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 566 459 B1

## Description

La présente invention a pour objet un réactif et un procédé utile pour cliver une fonction protégée, à l'occasion d'une synthèse organique.par un groupe alcoxy-carbonyle, d'avec celui-là. Elle vise plus particulièrement le clivage de tel groupe dont le groupe alcoxyle présente une insaturation en B tels que les groupes propargyle, benzyle ou allyle.

Il est courant de protéger une molécule en bloquant les fonctions qui dans les conditions opératoires envisagées seraient réactives ou réputées telles par des groupes qualifiés de protecteur.

Ces techniques sont particulièrement utiles lors des synthèses peptidiques et les fonctions les plus couramment protégées sont les fonctions acides, alcool, amines ou thiol.

Parmi les groupes les plus couramment utilisés, on peut citer le groupe BOC ou terbutyloxycarbonyle, les groupes Z ou benzyloxyle, voire même le groupe FMOC. il convient de signaler que les groupes de structure allylique auraient été considérés comme potentiellement très intéressants si l'on disposait de moyen de clivage adapté.

La déprotection usuellement utilisée est une lyse en milieu acide, en général en milieu halohydrique anhydre (c'est-à-dire avec une teneur en eau en général inférieure à 1%, avantageusement à $10^{-3}$, de préférence à $10^{-4}$).

Cette technique présente toutefois de nombreux inconvénients. La réaction de clivage est parfois lente ou nécessite de gros excès de réactif. Les groupements alcoxyles ont tendance à se transformer en carbocation avec évolution vers les doubles liaisons lorsque cela est possible, ou vers des réactions d'alcoylation sur le noyau, ce qui est particulièrement gênant dans le cas des synthèses de peptides dont la séquence présente des résidus nucléophiles tels que des noyaux aromatiques (tryptophane, tyrosine, phénylalanine,....) ou soufrés (méthionine). Ils peuvent même alcoyler les fonctions en cours de libération.

Cette technique ou n'est pas sélective, ou donne de mauvais résultats dans le cas des groupes alcoxycarbonyles présentant une insaturation en B.

C'est la raison pour laquelle on a proposé de réaliser le clivage des fonctions alcoxycarbonyles présentant les caractéristiques ci-dessus au moyen des métaux de la colonne VIII en général complexés par divers coordinants.

Toutefois bien que facilitant légèrement le clivage cette technique présente les mêmes inconvénients "mutatis mutandis" que ceux décrits précédemment en particulier alcoylation des fonctions nucléophiles présentes dans la molécule synthétisée.

*Pour pallier cette difficulté, on a associé des réducteurs puissants mais couteux pour donner le propène, par exemple dans l'article du JOC 52, 1987. pages 4984-4993, où l'on propose de réduire le groupe allylique en propène sous l'action d'hydrure de tributylétain en présence de palladium complexé par triphenyl phosphine.*

*Par ailleurs dans EPA 407 256, l'utilisation de carbonate d'éthyle et d'allyle substitué ou d'acétate allyle substitué avait déjà été décrite comme un système capable d'alcoyler divers nucléophiles.*

C'est pourquoi, un des buts de la présente invention est de fournir un procédé et un réactif qui accélèrent sensiblement la cinétique de clivage.

Un autre but de la présente invention est de fournir un procédé et un réactif qui évitent les réactions d'alcoylation des noyaux aromatiques.

Un autre but de la présente invention est de fournir un procédé et un réactif qui évitent les réactions d'alcoylation des fonctions qualifiées de nucléophile.

Ces buts et d'autres, qui apparaîtront par la suite, sont atteints au moyen d'un réactif utile pour cliver les fonctions alcoyloxycarbonyles insaturées.

Un autre but de la présente invention est de fournir un procédé de clivage qui soit sélectif tant vis à vis des divers groupes allyles que vis à vis des autres groupe bloquant.

Ce clivage qui a lieu entre le groupe alcoyle [dans la présente description ALCO-*yle* est pris dans son sens étymologique de reste hydrocarboné d'un ALCO-*ol* après ignorance de la fonction alcool (ou ol)] et la fonction carbonyle (-CO-) peut entraîner d'autre rupture de liaison parachevant ainsi la libération de certaines fonctions.

Le réactif selon la présente invention comporte :

a) une phase aqueuse ;
b) un catalyseur comportant au moins un élément de la colonne VIII de la classification périodique des éléments, ledit élément de la colonne VIII de la classification périodique étant maintenu dans la phase aqueuse par complexation avec au moins un agent de coordination hydrosoluble ;
c) un composé nucléophile hydrosoluble.

Lorsque le substrat et/ou le produit d'arrivée sont peu solubles en phase aqueuse, il est possible de mener la réaction, ou bien en ajoutant un tiers solvant B, ou bien en un système multiphasé, soit sans adjonction, soit en ajoutant un solvant A, soit en ajoutant simultanément un tiers solvant B et solvant A.

Les solvants A sont des solvants organiques choisis de manière qu'ils dissolvent au moins 1 %, avantageusement au moins 2 %, de préférence 5% en masse du substrat et sont suffisamment hydrophobes pour n'être pas miscibles à l'eau en toute proportion.

Il est préférable que l'eau ne puisse dissoudre qu'au plus 10 % de solvant A, avantageusement au plus 1 %, en masse et ce même en présence du substrat en tant que tiers solvant.

Il est préférable que le solvant A ne puisse dissoudre qu'au plus 10 % d'eau, avantageusement au plus 1 %, en masse et ce même en présence du substrat en tant que tiers solvant.

Les solvants A peuvent être des mélanges, y compris des fractions pétrolières. Naturellement, dans les conditions opératoires les solvants A doivent être inertes vis-à-vis des substrats et des réactifs utilisés.

Les familles préférées de solvants sont choisies dans le groupe constitué par les hydrocarbures, les dérivés aromatiques, les éthers, les esters et les solvants halogénés.Si l'on désire récupérer ces solvants il est souhaitable qu'ils soient moins nucléophiles que ledit composé nucléophile, de manière à ne pas interférer avec la réaction à moins, bien sur que ledit composé nucléophile soit en excès suffisant pour jouer un rôle de (tiers)solvant.

A titre de paradigmes de ces familles, on peut citer comme dérivés aliphatiques halogénés le dichlorométhane, le dichloro-1,2-éthane, le trichloro-1,1,1-éthane, comme dérivés aromatiques le toluène et comme dérivés aromatiques halogénés le chlorobenzène, comme esters l'acétate d'éthyle et l'acétate d'isopropyle, comme éthers, l'oxyde de tertiobutyle et de méthyle ainsi que l'anisole et les alcools lourds, c'est-à-dire répondant aux contraintes d'immiscibilité comme spécifié ci-dessus.

Pour des raisons d'économie industrielle, il est préférable que le solvant A soit distillable sous pression atmosphérique ou sous vide primaire ou secondaire.

Parmi les solvants A il convient de citer particulièrement ceux qui sont phénoliques et qui sont détaillés dans les demandes Françaises de la demanderesse FR-A-2 655 348 et FR-A-2 682 376.

Selon un mode de réalisation de la présente invention, lorsque les substrats ne sont pas hydrosolubles, il est alors loisible à l'homme de métier de rajouter un tiers solvant B dont le rôle sera de solubiliser le substrat dans la phase aqueuse.

Ce tiers solvant B pourra se partager entre phases aqueuse et organique lorsque cette dernière existe, soit initialement, soit du fait de l'éventuel emploi simultané du solvant A.

Il est préférable que l'eau puisse dissoudre au moins 1/10 de tiers solvant B, avantageusement au moins 1/3, en masse et ce même en présence du catalyseur avec ses agents de coordination.

Avantageusement, on ajoute le tiers solvant en quantité suffisante pour que la quantité de substrat soluble dans la phase aqueuse soit au moins du même ordre de grandeur que la quantité de catalyseur présent dans la phase aqueuse en début de réaction.

Parmi les solvants utilisables comme tiers solvant, on peut citer les solvants hydrosolubles de types alcool, nitrile, éther (surtout cyclique), acide, sulfone, sulfoxyde, amide simple ou polyfonctionnel (comme l'urée), ester, cétone, voire amine notamment dans le cas où ledit composé nucléophile sert aussi de tiers solvant.

Selon l'invention la notion de phase aqueuse doit-être prise au sens large, en effef il n'est pas nécessaire que, outre le tiers solvant, le système catalytique et les constituants spécifiés dans le corps de la présente description, il y ait une proportion importante d'eau, on peut obtenir de bon résultats même avec des quantités d'eau aussi faible que 5% en volume, voire inférieure à 1 %.

Certains résultats excellents sont obtenus sans addition spécifique d'eau, l'eau présente dans les solvants non séchés s'étant révélée suffisante.

Plus précisément, plutôt que de parler de phase aqueuse lato sensu, il serait plus conforme de se référer à une phase hydrophile ayant des propriétés de dissolution et de solvatation hydroïdes (c'est à dire qui ressemble à l'eau).

Ainsi on ne s'écartera pas de la présente invention en utilisant des phases hydrophiles (c'est-à-dire comportant comme constituant principal un solvant, ou un mélange de solvant, miscible en toute proportion et elle-même miscible en de larges proportions) ayant la propriété de dissoudre un système catalytique de type spécifié ci dessus hydrosoluble (c'est à dire soluble dans l'eau sensu stricto dans tout ou partie du domaine de concentration prévu dans la présente invention).

Comme on l'a vu à propos du phénol F , on peut également choisir le solvant A de manière qu'il joue également le rôle de tiers solvant B (ou réciproquement). Dans ce cas, on utilise des solvants présentant une fonction polaire du type de celle des tiers solvants et ayant une chaîne lipophile choisie de manière que l'eau dissolve ledit tiers solvant à raison d'environ 1/100 à 1/10 en masse.

Les métaux donnant les meilleurs résultats catalytiques sont les métaux de la mine du platine, de preférence ceux qui sont isoélectroniques du palladium et à l'état de valence qui soit isoélectronique du palladium zéro ; toutefois, il peut être économiquement intéressant d'utiliser des métaux moins lourds en raison de leur coût bien moindre ; au sein de la famille des métaux de la mine du platine, chacun d'entre eux présente des spécificités qui les rendent plus ou moins intéressants selon les cas ; le palladium, notamment à l'état d'oxydation zéro donne le plus souvent les meilleurs résultats.

Avantageusement lesdits agents de coordination sont des dérivés trivalents hydrocarbonés des éléments de

la colonne VB, avantageusement de période d'un rang supérieur à la deuxième et en général inférieure à la sixième, de la classification périodique des éléments (supplément au Bulletin de la Société Chimique de France Janvier 1966 N°1). Outre ceux qui sont détaillés par la suite on peut donner comme exemples de tels composés les dérivés des acides oxygénés trivalents (phosphoreux, arsénieux, antimonieux et pour mémoire nitreux) dérivés obtenus notamment par estérification ou par substitution d'au plus deux des trois oxhydryles (la trisubstitution conduit en fait aux pnictines qui fait l'objet d'une description plus détaillée).

Parmi les dits dérivés hydrocarbonés des éléments de la colonne V les préférés sont ceux qui dérivent des pnictures d'hydrogène par substitution totale ou partielle de l'hydrogène par des restes hydrocarbonés qui peuvent être reliés à l'atome de la colonne V B par une double (comme dans les imines) ou une triple liaison (comme dans les nitriles).

Toutefois les dérivés hydrocarbonés des éléments de la colonne V dérivent avantageusement des pnictures d'hydrogène par substitution totale ou partielle de l'hydrogène par des restes hydrocarbonés monovalents avantageusement par des alcoyles [dans la présente description ALCO-*yle* est pris dans son sens étymologique de reste hydrocarboné d'un ALCO-*ol* après ignorance de la fonction alcool (ou ol)]; ces composés alcoylés seront, par analogie avec le terme de pnicture, désignés dans la présente description, sous le terme de pnictines..

Ainsi dans le cas de l'azote la substitution du nitrure d'hydrogène (ammoniac) donne les amines, dans le cas du phosphore la substitution du phosphure d'hydrogène donne les phosphines, dans le cas de l'arsenic la substitution de l'arséniure d'hydrogène donne les arsines et dans le cas de l'antimoine la substitution de l'antimoniure (ou stibiure) d'hydrogène donne les stibines. Ils sont avantageusement choisis parmi les dérivés hydrocarbonés du phosphore tels que les phosphines.

Avantageusement ledit catalyseur comporte comme agent de coordination hydrosoluble une pnictine, une trialcoylphosphine, de préférence (pour des raisons économiques) une triarylphosphine, en général une triphénylphosphine. Ladite phosphine et ledit métal de la colonne VIII sont avantageusement sous forme de tétrakis phosphine métal.

Pour rendre solubles les agents de coordination et notamment les pnictines, il convient de greffer des groupes hydrosolubilisants polaires.

On peut greffer des groupements neutres tels que les polyols, mais compte tenu de la forte lipophilicité des pnictines, il est préférable que les groupes greffés soient ioniques, cationiques comme les ammoniums quaternaires ou anioniques, comme tout groupe constituant la base associée des acides de préférence forts. Dans ce dernier cas, on peut citer les groupes carboxyliques sulfoniques, phosphoniques, et plus généralement ceux donnant une hydrophilie équivalente.

On peut notamment citer les groupes utilisés pour modifier les phosphines pour obtenir celles visées dans le brevet français déposé sous le n° 76/22824 et publié sous le n° 2.366.237 ou dans le brevet français publié sous le n° 2.549.840.

A titre de paradigme des phosphines hydrosolubles, on peut citer les triphénylphosphinetrisulfonates $P(C_6H_4-SO_3^-)_3$ solubles, par exemple alcalins et celles de formule $P(C_6H_4-CO_2H)_3$, de préférence sous forme anionique.

Ainsi selon une mise en oeuvre particulièrement intéressante de la présente invention, on peut utiliser un système biphasique dans lequel une des 2 phases liquides est une phase aqueuse, dans laquelle le métal de la colonne VIII est solubilisé en phase aqueuse par une pnictine, ou équivalent, hydrosoluble.

Lorsqu'il y a des risques d'empoisonnement du catalyseur, c'est à dire lorsque on utilise des nucléophiles dits "mou", en général dont la fonction nucléophile est à base de métalloïdes de rang élevé au moins égal à celui du phosphore ou du soufre, il est préférable d'utiliser:

- soit des phosphines dont la basicité est élevée (la basicité qui est faible avec les triarylphosphines, croît avec le nombre de remplacements d'aryle par des chaînes dont les éventuelles insaturations [leur présence n'est pas souhaitable] sont non conjuguées avec les doublets de l'élément de la colonnes V), comme par exemples les bi- ou tri cyclohexylphosphines;
- soit des pnictines polyfonctionnelles, en général bifonctionelle, permettant une chélation du métal par les fonctions pnictines; en général les fonctions pnictines sont, en prenant le chemin le plus direct, séparées par 2, 3 ou 4 atomes, le plus souvent de carbone ; les formules du type $\omega,\omega$'diphenylphosphinoéthane; ou $\omega,\omega$'diphenyl-phosphinobutane.

Cette technique facilite grandement la récupération et le recyclage du catalyseur, lequel recyclage est un des paramètres clés de la rentabilité de ce type de procédé en raison du prix toujours accru des métaux de la mine du platine.

Dans le cadre de la présente invention, on peut utiliser un catalyseur métallique sous forme élémentaire (degré d'oxydation zéro) ou sous forme oxydée. Ces catalyseurs peuvent se présenter sous forme de sels, d'oxydes ou de complexes. Parmi les sels, oxydes et complexes des métaux précédemment cités, à l'état d'oxydation II, on peut mentionner les chlorures de palladium, l'acétate de palladium, le chlorure de palladium complexé par le benzonitrile. Il convient de souligner que les anions sont de faible importance, seuls les cations comptent.

Parmi les complexes des métaux à l'état d'oxydation zéro, on peut citer le palladium dibenzylidèneacétone.

Il convient de souligner que le niveau d'oxydation du métal n'est pas nécessairement conservé dans le réacteur en effet les pnictines sont en général assez réductrice pour réduire le palladium à l'état élémentaire même introduit sous forme palladeuse.

Pour une meilleure mise en oeuvre de l'invention, on préfère utiliser une quantité de catalyseur telle que le rapport molaire entre le catalyseur métallique et les composés des éléments de la colonne V, lorsque ces derniers composés sont sous forme d'agents de coordination, trop souvent désignés sous l'expression anglo-saxonne de Ligand, soit compris entre 2 et 100, plus généralement de 4 à 30. Ces rapports molaires doivent tenir compte du nombre de fonctions coordinantes par molécule ; ainsi lorsqu'on utilise comme agent de coordination des molécules ayant deux fonctions pnictine, il convient de diviser par deux les valeurs des domaines ci-dessus.

La quantité de phase aqueuse utilisée est telle que la concentration du métal de la colonne VIII soit de préférence supérieure à $10^{-5}$, avantageusement de $10^{-2}$ à $10^{-3}$ M dans le solvant.

Ledit composé nucléophile doit présenter deux caractéristiques; il doit d'une part être nucléophiles, c'est-à-dire riches en électrons.et d'autre part être hydrosoluble

Dans la présente demande on préférera les ceux qui sur diverse échelles de nucléophilies en ont une supérieure à celle de l'ammoniac ( voir le March 3ème édition pp 307 309 )

En fait le choix du nucléophile dépend des fonctions à déprotéger en général on préfère utiliser comme nucléophiles, des molécules porteuses de fonction(s) au moins aussi nucléophile que les fonctions vis-à-vis desquelles il convient d'être sélectif.

Lorsque les réactifs s'y prêtent, selon une mise en oeuvre particulièrement intéressante de la présente invention, on protone les fonctions dont l'alcoylation est à éviter et on choisit un nucléophile non protonable dans les conditions opératoires. Dans cette hypothèse la contrainte ci-dessus sur le caractère nucléophile devient que la nucléophilicité doit être supérieure à celle de l'ion $NH_4^{+}$· cette protonation se fait en phase aqueuse au moyen d'acide dont le pKa est inférieur d'au moins 1 point de préférence d'au moins 2 points au pKa de l'acide associé à la fonction nucléophile que l'on désire protéger. Un excès d'au moins 10 % par rapport à la quantité nécessaire à la neutralisation est préférable

Ces nucléophiles peuvent être des anions ou de molécules neutres. Pour illustrer la richesse de cette catégorie de substrat, on peut citer de façon non limitative :

- les sulfures et disulfures organiques aliphatiques (primaires, secondaires ou tertiaires), aromatiques ou hétérocycliques,
- les thiols,
- les pnictines, (amine, phosphine,.......) de préférence secondaires

Pour être hydrophile,ou plutôt hydrosoluble, c'est à dire qu'il soit soluble dans l'eau, ledit composé nucléophile doit être tel que dans les conditions normales l'eau soit capable d'en dissoudre au moins 0, 2 avantageusement 0,5 de préférence 1 équivalent gramme de fonction nucléophile.

Il est à noter que l'on peut rendre hydrosolubles des réactifs nucléophiles qui ne le seraient pas ou peu eu prévoyant une fonction fortement hydrophile sur la molécule. les fonctions acides forte ou moyenne (pKa au plus égal à 6, avantageusement à 5, de préférence à 4), qu'elles soient sulfurées,(sulfonique, monoester sulfurique,....), phosphorées (ester phosphorique, acide phosphonique, acide phosphinique,.....), carbonées, ou autres, donnent des résultats suffisamment bons pour que l'on se demande s'il n'y a pas de synergie. Les meilleurs résultats sont à ce jour obtenus avec une fonction carbonée à savoir la fonction carboxylique.

Ainsi, avantageusement, les résultats obtenus à partir de nucléophiles où la fonction nucléophile est portée par un carbone postvicinal ou de préférence vicinal de celui portant la fonction acide. l'un des meilleurs nucléophiles est donc l'acide thiosalicylique (H-S Φ-COOH), notamment sous forme acide ou monoanionique.

Il est, bien sur, préférable que l'eau et le nucléophile soit miscible en toute proportion. un même composé peut porter plusieurs fonctions nucléophiles

Il est préférable qu'il y ait, rapporté à au nombre de carbone au moins une fonction nucléophile pour 10 atomes de carbone avantageusement une pour 8 de préférence une pour 4.

Il est également préférable d'utiliser de petites molécules dont le nombre de carbone ne soit pas significativement supérieur à la dizaine.

Enfin il peut être pratique de choisir un composé nucléophile qui voit sa solubilité décroître considérablement avec la température pour passer en phase gazeuse permettant ainsi un déplacement aisé par distillation.

En général ledit composé nucléophile est présent (initialement mais plus préférablement en fin de réaction) à une concentration d'au moins 1/2, avantageusement 2, de preférence 5 équivalent gramme par litre.

Lorsque le composé nucléophile présente ramenés à une fonction nucléophile une masse moléculaire faible

des concentration aussi élevées que 10 équivalents par gramme sont souvent dépassée.

Lorsque la sélectivité apportée par l'utilisation de la phase aqueuse n'est pas jugée suffisante et que l'on désire l'augmenter, on peut pour ce faire jouer sur l'excès dudit composé nucléophile par rapport au substrat, par exemple en augmentant l'excès stoechiométrique (en général largement supérieur à 10%) par rapport à la réaction désirée pour le porter à une valeur au moins égale à 1/4, avantageusement à 1/2 de préférence à un fois de la quantité stoechiométrique (c'est à dire travailler avec des quantités respectivement au moins égales à 5/4 ; 3/2 et 2 Q.S.).

Ainsi, avantageusement selon la présente invention, la quantité du dit nucléophile est au moins égale à 3/2 fois la quantité stoechiométriquement nécessaire.

Un autre but de la présente invention est de fournir un procédé qui accélère sensiblement la cinétique de clivage.

Un autre but de la présente invention est de fournir un procédé qui évite les réactions d'alcoylation des noyaux aromatiques.

Un autre but de la présente invention est de fournir un procédé qui évite les réactions d'alcoylation des fonctions qualifiées de nucléophile.

Ces buts et d'autres, qui apparaîtront par la suite, sont atteints au moyen d'un procédé de traitement de molécule comportant au moins une fonction alcoyloxycarbonyle insaturée où ladite molécule est soumise aux réactifs spécifié ci dessus

Avantageusement lesdites molécules comportant au moins une fonction alcoyloxycarbonyle répondent à la formule suivante(I) :

$$Z\text{-}O\text{-}C(R_1)(R_2)\text{-}C(R_3)\text{==}C(R_4)(R_5) \qquad \text{(III)}$$

où

- $R_1$ représente un hydrogène ou un radical alcoyle, de préférence à 1 ou 2 atomes de carbone ;
- $R_2$ représente un hydrogène ou un radical alcoyle, de préférence à 1 ou 2 atomes de carbone ;
- $R_3$ représente un hydrogène ou un radical alcoyle, de préférence à 1 ou 2 atomes de carbone, ou, avec $R_4$ forme une double liaison supplémentaire ;
- $R_4$ représente un hydrogène ou un radical alcoyle, de préférence à 1 ou 2 atomes de carbone ou, avec $R_3$ forme une double liaison supplémentaire ;
- $R_5$ représente un hydrogène ou un radical alcoyle , de préférence à 1 ou 2 atomes de carbone ou aryle;
- Z un radical acyle y compris carbamyle alcoxycarbonyle thioalcoxycarbonyle;
- Z contient la molécule protégée et qu'il faut libérer de son groupe protecteur.

$R_5$ peut être un groupe dit "Ar" tel que décrit dans la demande de brevet britannique déposée le 31.12.1990 sous le N°90 28208.8 intitulée "groupe Protecteur" et publiée sous le N° 2.251.242

$R_5$ et $R_4$ peuvent être des fractions du groupe dit "Ar" dans la demande ci-dessus de manière que $R_5$ et $R_4$ ainsi que le carbone qui les porte forme un radical ar tel que défini dans la demande britannique ci dessus.

$R_5$ peut être tout groupe lipophile tel que décrit dans les demandes de brevets francais au nom de la demanderesse déposées le 02.10.1989 sous le numéro N°89/13054 (Publiée sous le N° 2 652 581) et intitulée "Procédé de solubilisation de peptides et procédé de synthèse de peptides." et celle déposée le 04.12.1989 sous le N°89/15957 (Publie sous le N° 2 655 348) et intitulée "Milieu réactionnel et solubilisant de peptides et procédé de synthèse utilisant ce milieu."

D'une manière générale l'association du groupe désigné par Ar dans la demande britannique ci-dessus avec un groupement allyloxy carbonyle en temps que "L" est très favorable.

Le plus souvent Z est de formule Z'-CO- avec Z' étant le radical issu de la molécule à protéger la liaison remplaçant un hydrogène de la fonction dont on désire la protection.

Z comporte un nombre de carbone supérieur à 3, le plus souvent à 5, et fréquemment à 10.

Les molécules protégées sont, ou présentent comme chaînons, souvent des acides aminés et des peptides, des sucres et notamment des nucléotides.

Z' est en général polyfonctionnel (en général au moins bi-, plus généralement au moins trifonctionnel)les dites; fonction étant le plus souvent protégé par divers groupe protecteurs il est souvent très important que la lyse d'une fonction protectrice soit sélective vis à vis des autres fonctions protectrices.ce qui est le cas du réactif selon la présent invention.

Ainsi la présente invention fournit un procédé utile pour le clivage sélectif d'une molécule dont au moins une fonction est protégée par un groupe protecteur allyloxycarbonyle et dont au moins une fonction est protégée par un groupe protecteur distinct du précédent (soit parce que le groupe allyle est distinct du précédent soit qu'il soit d'une autre nature). il s'agit donc d'un procédé de clivage sélectif tant vis à vis des divers groupes allyles que vis à vis des

autre groupe protecteur.

Il est préférable que Z' soit de structure Z"-$\chi$- avec $\chi$ étant un atome autre que de carbone, avantageusement des colonnes V ou VI.

Le terme alcoyle est pris dans son sens étymologique déjà spécifié avec la précision supplémentaire qu'il peut également signifier un groupement aryle.

Il est préférable qu'au moins 2, avantageusement 3, de préférence 4 des radicaux R1 à R5 soient au plus de deux carbones ; toutefois, au moins un des radicaux R1 à R5 peut être tel que l'alcool allylique soit un alcool lourd, par exemple de série aromatique, de type terpénique ou de série stéroïdique.

Ainsi, au moins 1 radical et au plus 3 radicaux R1 à R5 peuvent être des radicaux aryles polycycliques, condensés ou non, homo ou hétérocycliques.

selon un aspect des plus surprenants, la présente invention permet de rendre sélective la libération des fonctions protégées par des allyloxy carbonyles dont les substituants $R_1$ à $R_5$ sont différents : moins le groupement allylique est substitué, plus la libération est aisée. Plus précisément la réactivité des différents groupes allyles dépend fortement du degré de substitution du groupe allyle. Si l'on désigne par p le nombre de substituants $R_1$ à $R_5$., plus p est élevé, plus la sensibilité au réactifs à base de palladium zéro, ou d'autre métaux de la colonne VIII qui sont isoélectronique, décroît.

Un milieu monophasique distingue légèrement moins nettement les non substitués des monosubstitués mais distingue aisément les disubstitués des mono.

Enfin tout se passe comme si il y avait des seuils d'efficacité de la concentration limite basse du catalyseur différent selon le degré de substitution du groupement allyle, il est ainsi possible de réaliser des libération extrêmement sélective.

Cette réactivité différentielle entre les différents allyles peut conduire à des sélectivités totales en jouant sur les paramètres déjà évoqués : ainsi un milieu biphasique favorise très fortement les allyles peu ou non substitués.

Cette remarquable propriété permet la synthèse de molécules complexes telles les peptides et les (poly)nucléotides en protégeant par allyloxycarbonyle; des fonctions dont la libération doit être étagée.

Ainsi le présente invention permet d'utiliser des molécules de structure :

$\zeta$ (-CO-O-Allyl$_i$)$_n$

avec i prenant toute les valeurs entières de 1 à n (n étant un nombre entier au moins égal à 2, de préférence à 3 et en général au plus égal à 100, voire à 50)

-allyl$_i$ étant de formule

-C($R_1$)($R_2$)-C($R_3$)==C($R_4$)($R_5$)

$\zeta$ étant le reste de la molécule polyfonctionelle

Les allyl$_i$ étant avantageusement tels qu'il y ait dans la molécule au moins deux, de préférence 3, formules différentes de allyl$_i$. Il est préférable que ces formules présentent respectivement 0, 1 et /ou 2 substituants $R_1$ à $R_5$.

La température réactionnelle est en général comprise entre le point de fusion finissante et le point d'ébullition commençante du milieu réactionnel. avantageusement entre 0°C et 100°C de préférence entre l'ambiante (environ 20°C) et 50°C.

Il apparaît que la sélectivité croît lorsque la réaction est menée dans deux phases mais la cinétique, bien que restant en général élevée, décroît.

Le procédé selon l'invention respecte la géométrie des molécules et est de ce fait particulièrement bien adapté à la chimie des molécules chirales.

Les exemples non limitatifs suivants illustrent l'invention.

__Définitions__

TT = Taux de Transformation :

$$TT = \frac{\text{nombre de moles de produit transformé}}{\text{nombre de moles de produit initial}}$$

RR = le rendement sur le produit initial

$$RR = \frac{\text{nombre de moles de produit final}}{\text{nombre de moles de produit initial}}$$

RT = le rendement sur le produit transformé

$$RT = \frac{\text{nombre de moles de produit final}}{\text{nombre de moles de produit transformé}}$$

EXEMPLES : MODES OPERATOIRES GENERAL DES EXEMPLES PROTECTIONS D'AMINES PRIMAIRES OU SECONDAIRES

Dans un ballon, sous argon, l'amine (1 éq) est dissoute dans du THF anhydre 10 ml pour 1 g). Le mélange est porté à 0°C, puis le chloroformiate d'allyle (0,5 éq) est additionné goutte à goutte pendant 5 minutes. Après 45 minutes le milieu est porté à température ambiante, la réaction est suivie en CCM (chromatographie sur couche mince).
Après 12 heures, le précipité est filtré à l'éther sur célite. La phase organique est lavée 3 fois par 20 ml $H_2O$ ($NaHCO_3$/HCl 5 % /$H_2O$). La solution éthérée est séchée sur $MgSO_4$ puis condensée sous pression réduite. Les produits protégés sont chromatographiés sur gel de silice ou distillés.

PROTECTIONS D'ACIDES

Dans un ballon, sous argon, est introduit l'acide (1 éq) dans l'acétonitrile, le THF ou le $CH_2Cl_2$ anydre (10 ml pour 1g). Le mélange est porté à 0°C. Le DBU (1,2 éq) puis le dérivé bromé (1,2 éq) ou le chloroformiate d'allyle (1,2 éq) sont alors additionnés. Après 30 minutes le milieu réactionnel est porté à température ambiante.

La réaction est généralement rapide, de l'ordre de quelques heures ; elle est suivie en CCM. Le milieu réactionnel est évaporé sous pression réduite. Le résidu est repris avec de l'eau et extrait par 3 x 35 ml d'acétate d'éthyle. Les fractions organiques réunies sont séchées sur $MgSO_4$ puis évaporées sous pression réduite pour donner des huiles jaunes. Les produits sont purifiés par chromatographie sur gel de silice ou distillés.

PROTECTIONS D'ALCOOLS

Dans un ballon, sous argon, sont introduits l'alcool 1 éq et le THF anydre (10 ml pour 1 g). Le mélange est porté à 0°C et 3 équivalents de pyridine sont additionnés suivie de 1,5 à 3 éq de chloroformiate d'allyle (goutte à goutte). Après 30 minutes, le milieu réactionnel est porté à température ambiante, la réaction est suivie en CCM. Après 12 heures, le milieu réactionnel est évaporé sous pression réduite. Le résidu est repris à l'eau et extrait par 3 x 30 ml d'éther ou d'acétate d'éthyle. Les fractions organiques sont réunies et séchées sur $MgSO_4$ puis évaporées sous pression réduite pour donner des huiles jaunes. Les produits sont purifiés par chromatographie sur gel de silice ou distillés.

DEPROTECTION EN MILIEU AQUEUX (MONO OU BIPHASIQUE)

Mode opératoire général :

Dans un tube, sous argon, sont introduits le produit à déprotéger et le solvant (3 ml pour 100 à 500 mg de réactif). Le nucléophile (la diéthylamine 1,2 à 15 éq) est ajouté ainsi que l'eau (0,3 à 0,5 ml). On ajoute ensuite le système catalytique (2,5 à 5%) : Pd $(OAc)_2$ + TPPTS (1:2).

Le milieu réactionnel est ensuite agité vigoureusement en faisant attention à l'homogénéité du milieu. Après réaction (généralement très rapide de 5 à 15 minutes), le milieu réactionnel est filtré sur gel de silice avec un mélange (AcOEt/cyclohexane, 1:1). Le filtrat est évaporé sous pression réduite et le produit déprotégé peut être purifié par chromatographie sur gel de silice ou distillé.

Dans le but d'un recyclage ou d'une réaction excédant la demi heure, il faut dégazer le solvant, l'eau et la diéthylamine.
Les solvants peuvent être : $CH_3CN$ (pour un milieu homogène) : RCN, AcOEt (pour un milieu biphasique).

DEPROTECTION EN MILIEU FAIBLEMENT AQUEUX

Mode opératoire général :

Le catalyseur est préformé en agitant 15 minutes, sous argon, le $Pd(dba)_2$ et le coordinant diphénylphosphinobutane (D.P.P.B.) (1:1) dans 1 ml de THF anydre (2,5 à 5 % de catalyseur pour la réaction). On obtient alors une solution brun-roux.

Dans un tube, sous argon, sont introduits le substrat à déprotéger (100 à 500 mg) et 3 ml de THF anhydre.

Puis sont additionnés le nucléophile (composé soufré) ou la diéthylamine (dégazée), et enfin le système catalytique préformé.

Lorsque le nucléophile est la diéthylamine, le milieu réactionnel est filtré sur gel de silice (éluant : AcOEt/cyclohexane (1:1). Le filtrat est ensuite concentré sous pression réduite.

Lorsque le nucléophile est l'acide mercapto-2-benzoïque, ce dernier peut être séparé du produit déprotégé par traitement avec une solution basique (NaOH 10 %) au cours d'une extraction à l'acétate d'éthyle. La phase organique est séchée puis concentrée. Les produits déprotégés peuvent être purifiés par chromatographie sur gel de silice ou distillés.

RECYCLAGE EN MILIEU AQUEUX BIPHASIQUE

Dégazage impératif des solvants et du nucléophile.

Mode opératoire :

Losque la réaction de déprotection est terminée, il nous est alors possible de recycler le catalyseur en le canulant (au moins 10 fois). Le recyclage peut être décrit de la manière suivante entre deux tubes à réactions : le premier contient le milieu biphasique : $C_3H_7CN$ (où nous trouvons le produit déprotégé, la diéthylamine en excès et le nucléophile alcoylé) et l'eau (dans laquelle est présent le système catalytique), le deuxième quant à lui ne contient que la phase organique composée de $C_3H_7CN$, du nouveau produit à déprotéger et de la diéthylamine. Le canulage peut se faire du premier tube au second en poussant la phase aqueuse du premier réacteur dans le deuxième. Le recyclage doit se faire sous argon avec des réactifs et des solvants totalement dégazés. La phase aqueuse se canule d'autant plus facilement qu'elle se trouve sous la phase organique.

**MODE OPERATOIRE DES EXEMPLES 1 A 30**

Substrat protégé → Substrat libéré

Dans un réacteur, on introduit environ 250 mg [(n = 0,0012 mole de substrat protégé par la fonction "Alloc" (= allyloxy carbonyle)] que l'on dissout dans 3 ml de milieu réactionnel (comme par exemple $CH_3CN$). Après purge et dégazage à l'argon , le nucléophile (diéthylamine) est ajouté (2,2 éq. ; n =0,0026 mole ; v = 0,272 ml) sous agitation et Argon.

On ajoute alors au milieu réactionnel :

- $6,2\ 10^{-3}$ g ((n = $2,76\ 10^{-5}$ mole) de Pd $(OAc)_2$ ;
- $8,12\ 10^{-2}$ g d'une solution aqueuse de TriPhénylPhosphinetriSulfonate (TPPTS) de sodium (solution aqueuse 32,5 % ; 0,505 milliéquivalent de Phosphine/g)
- 0,2 ml d'eau.

Les réactions sont suivies en Chromatographie en phase gazeuse en utilisant comme colonne celle connue sous les dénominations HPI méthyl silicone-Grum dont les dimensions sont 5 m x 0,53 mm x 2,65 ou celle connue sous le nom de 8E 30 capillaire, traitement habituel, chromatographie ou cristallisation. Le brut réactionnel est usuellement traité par évaporation des solvants après reprise au toluène, suivie d'une cristallisation, d'une chromatographie ou distillation du brut sous pression réduite.

La structure des produits est vérifée par analyse et comparaison d'authentique(s) en RMN, IR Chromatographie gazeuse et observation du pouvoir rotatoire lorsque l'on traite des molécules chirales. Rendement exprimé en % de produit récupéré.

DEPROTECTION D'ALCOOLS

**Réactifs** = $Pd(OAC)_2$, TPPTS à Température ambiante dans $CH_3CN/H_2O$

| Exemples N° | Substrats | Produits obtenus | Temps en mn | Rendement | Rapport nucléophile substrat |
|---|---|---|---|---|---|
| 1 | Allyloxy-carbonate de benzyle | Alcool benzylique | 10' | 90 % | 2,2 |
| 2 | Allyloxy-carbonate de cyclohexyle méthyle | Cyclohexyl carbinol | 10' | 79 % | 2,2 |
| 3 | Allyloxy-carbonate de citronéllyle | Citronéllol | 5' | 94 % | 2,2 et 2,5 |
| 4 | $(tBu)(\Phi)_2Si-OCH_2-$*$CH(OAlloc)CH_2-COOMe$ | $(tBu)(\Phi)_2Si-OCH_2-$*$CHOHCH_2COO-Me$ | 10' | 98 % | 2,5 éq. |

| Exemples | Substrate | $HNET_2$ (eq.) | Temps Min ou H | Produit | Rendement (c) (%) |
|---|---|---|---|---|---|
| 5 | 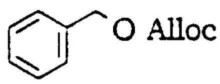 | 5 | 15' | 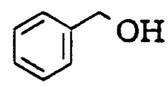 | 96 |
| 6 | 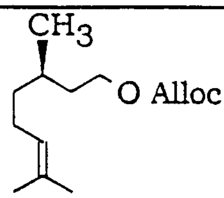 | 5 | 5' | 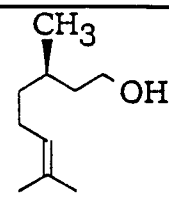 | 99 |
| 7 | 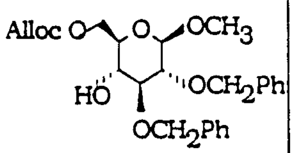 | 3,2 | 5 H | 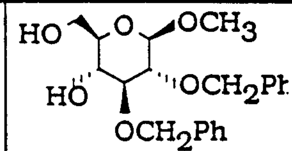 | 99 |
| 8 | 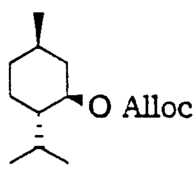 | 2 | 15' | 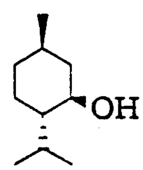 | 100(c) |
| 9 | 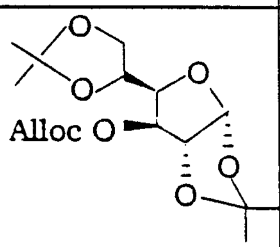 | 3,2 | 90' | 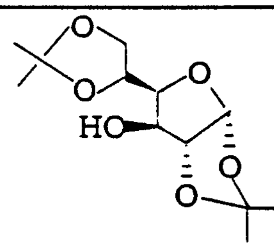 | 99 |
| 10 | 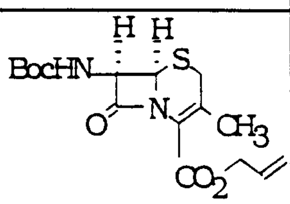 | 2,1 | 60' | 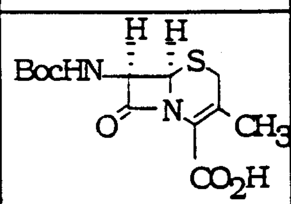 | 100(c) |

**DEPROTECTION D'ALCOOL**

**Réactifs** = $Pd(OAC)_2$, TPPTS à température ambiante biphasique

| **Exemples N°** | Substrats | Produits obtenus | Temps en mn | Rendement | Rapport nucléophile/substrat solvants |
|---|---|---|---|---|---|
| **11** | Allyloxycarbonate de citronéllyle | citronéllol | > 12 h | 51 % | 2,5 éq. $Et_2O/H_2O$ |
| **12** | " | " | 3h00 | 76 % | $CH_2Cl_2/H_2O$ 5 éq. |

A - Déprotection d'alcools

Etude dans différentes conditions en milieu biphasique de l'allyloxycarbonate de citronéllyle.

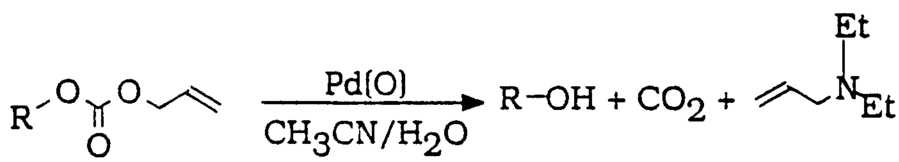


$Pd(O) = Pd(OAc)_2 + TPPTS\ (1/2)$

| Entrée | Substrats | Produits | Solvant | Temps (hrs)/RT | NuH (éq) | Rdt % |
|---|---|---|---|---|---|---|
| 13 | 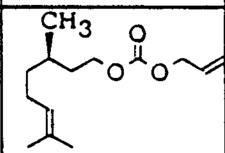 | 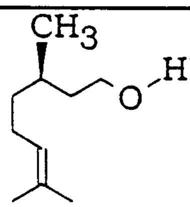 | $CH_3CN$ | 2,5 | HCOOH 3,5 | 51 % |

| Entrée | Substrats | Produits | Temps (mn) | Rdt (%) |
|---|---|---|---|---|
| 14 | 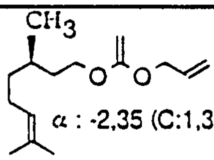 α : -2,35 (C:1,32; $CHCl_3$) | 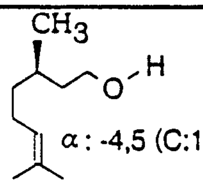 α : -4,5 (C:1 ; $CHCl_3$) | 5' | 94 |
| 15 | 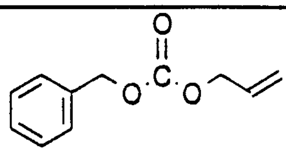 | 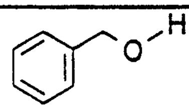 | 10' | 90 |
| 16 | 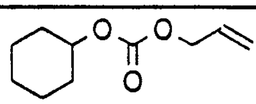 | 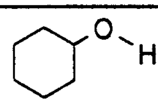 | 10' | 79 |
| 17 | 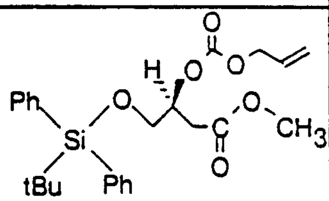 α: +16 (C:3,42 ; $CHCl_3$) | 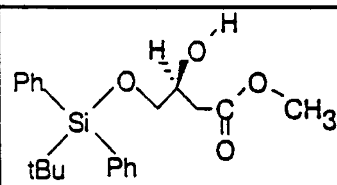 α: -7,65 (C:2,26 ; $CHCl_3$) | 10' | 98 |
| 18 | 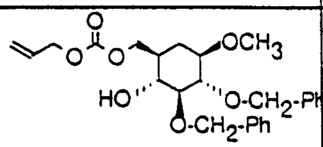 α: +21 (C:1 ; $CHCl_3$) | 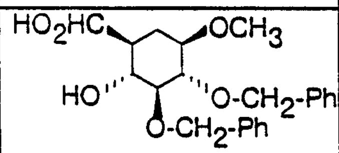 α: +19 (C:0,51 ; $CHCl_3$) | 20' | 99 |
| 19 | 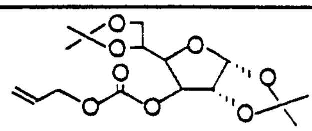 | 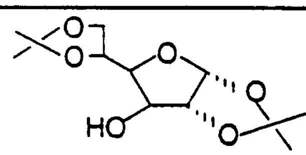 | 90' | 99 |

milieu : $Pd(OAc)_2$/ TPPTS (1:2) ; $CH_3CN/H_2O$ (6 : 1) à température ambiante.

EXEMPLE 20 : recyclage des catalyseurs

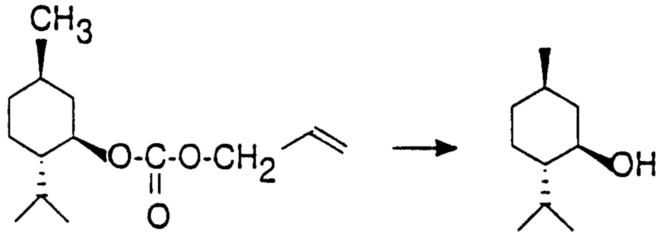

α: -66,4 (C:2 EtzO) α: -50 (C:10 EtOH)

| Nombre d' de la phase catalytique | Masse (g) | Nombre de mole ($10^{-3}$M) | Solvant* | Temps (rt) | Rdt - % (TT) |
|---|---|---|---|---|---|
| (1) | 0,2 | 0,83 | $C_3H_7CN/H_2O$ | 30' | 100 % |
| (2) | 0,2 | 0,83 | $C_3H_7CN/H_2O$ | 30' | 100 % |
| (3) | 0,2 | 0,83 | $C_3H_7CN/H_2O$ | 30' | 100 % |
| (4) | 0,20 | 0,83 | $C_3H_7CN/H_2O$ | 30' | 100 % |
| (5) | 0,2 | 0,83 | $C_3H_7CN/H_2O$ | 30' | 98 % |
| (6) | 0,2 | 0,83 | $C_3H_7CN/H_2O$ | 30' | 98 % |
| (7) | 0,2 | 0,83 | $C_3H_7CN/H_2O$ | 30' | 99 % |
| (8) | 0,2 | 0,83 | $C_3H_7CN/H_2O$ | 30' | 97 % |
| (9) | 0,2 | 0,83 | $C_3H_7CN/H_2O$ | 30' | 96 % |
| (10) | 0,2 | 0,83 | $C_3H_7CN/H_2O$ | 30' après 12h | 60 % (40 % non déprotégé) 98 % |

* solvant : $C_3H_7CN$, $H_2O$, $Et_2NH$ (2,2 eq) dégazé
(3 ml) (0,5 à 1 ml)
$Pd(OAc)_2$ ; TPPTS (5 %)

**DEPROTECTION D'ACIDES**

**Réactifs :** $Pd(OAC)_2$, TPPTS à température ambiante $CH_3CN/H_{2O}$ (monophasique)

| **Exemples N°** | Substrats | Produits obtenus | Temps en mn | Rendement | Rapport nucléophile substrat |
|---|---|---|---|---|---|
| **27** | 2,4 dichlorobenzoate d'allyle | Acide 2,4 dichloro benzoïque | 10' | 98 % | 2,2 éq |

(suite)

| Exemples N° | Substrats | Produits obtenus | Temps en mn | Rendement | Rapport nucléophile substrat |
|---|---|---|---|---|---|
| **28** | 2,4 dichlorobenzoate de cyclohexenyl | " " | 10' | 95 % | 2,2 éq. |
| **29** | phényl acétate d'allyle | acide phénylacétique | 15' | 99 % | 2,2 et 2,5 |

**DEPROTECTION D'AMINES PRIMAIRES**

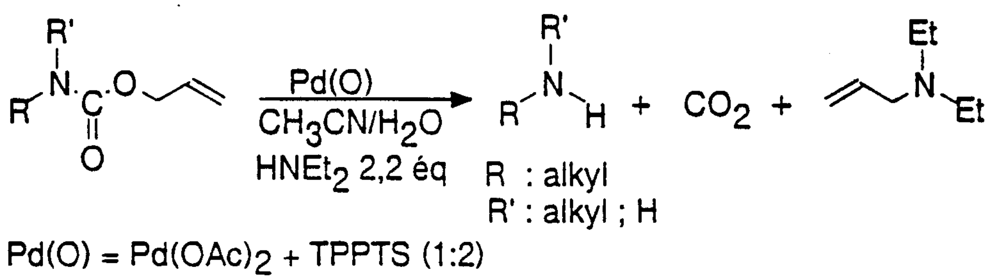


**Réactifs** : $Pd(OAC)_2$, TPPTS à température ambiante $CH_3CN/H_2O$

| Exemples N° | Substrats | Produits obtenus | Temps en mn | Rendement | Rapport nucléophile substrat |
|---|---|---|---|---|---|
| 30 | N-allyloxycarbonyl phénylalanine | Phénylalanine | 10' | 68 | NuH 2,2 éq. |
| 31 | N-allyloxycarbonyl benzylamine | Benzylamine | 9' | 72 | 2,2 éq. |
| 32 | N-allyloxycarbonyl α méthylbenzylique | α méthylbenzylamine | 5' | 85 | 2,2 éq. |
| 33 | N-allyloxycarbonyl iodo 3 aniline | Iodoaniline | 10' | 77 | 2,2 éq. |

| Entrée | Substrats | Produits | Temps (mn) | Rdt (%) |
|---|---|---|---|---|
| 34 | 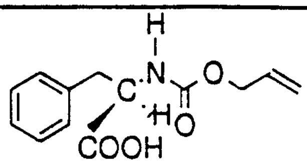 α: +14,5 (C : 0,4 ; $H_2O$) | 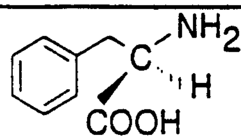 α: +35 (C : 2 ; $H_2O$) | 10' | 70 |
| 35 | 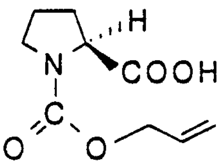 α: -84 (C:0,55 : $H_2O$) | 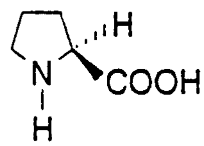 α: -71 (C:0,65 ; $H_2O$) | 15' | 90 |

milieu : $Pd(OAc)_2$/TPPTS (1:2) ; $CH_3CN/H_2O$ (6 :1)
$Et_2NH$ : 2,2 éq.

| Entrée | Substrats | Produits | Temps (mn) | Rdt (%) |
|---|---|---|---|---|
| 36 | 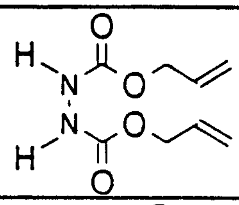 | $NH_2$-$NH_2$ | 10' | 99 |
| 37 | 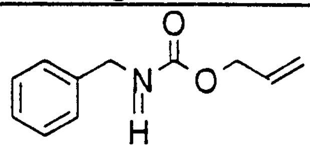 | 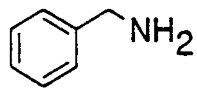 | 9' | 72 |
| 38 | 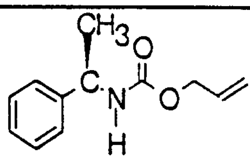 α: +61,52 (C:1,12 ; $CHCl_3$) | 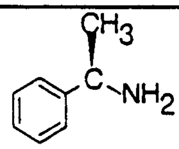 α: +29,56 (C:1,12 ; $CHCl_3$) | 5' | 85 |
| 39 | 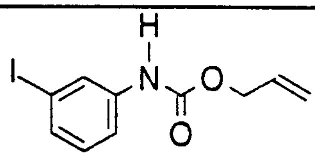 | 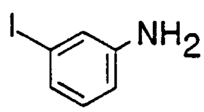 | 10' | 77 |

Milieu : $Pd(OAc)_2$/TPPTS (1:2) ; $CH_3CN/H_2O$ (6:1)
$Et_2N$ 2,2 éq.

**DEPROTECTION D'AMINES SECONDAIRES**

**Réactifs** : Pd $(OAC)_2$, TPPTS à température ambiante

| Exemples N° | Substrats | Produits obtenus | Temps en mn | Rendement | Rapport nucléophile/ substrat |
|---|---|---|---|---|---|
| 40 | NN'allyloxycarbonyl méthylbenzylamine | N-méthyl, benzylamine | 60 | 93 | 2,2 éq. AcOEt/$H_2O$ |
| 41 | NN'allyloxycarbonyl méthylbenzylamine | N-méthyl benzylamine | 15 | 82 | 2,2 et 4 $C_3H_7CN/H_2O$ |
| 42 | N-allyloxycarbonyl morpholine | Morpholine | 5 | 98 | 2,2 et 4,4 éq. $CH_3CN/H_2O$ |
| 43 | N-allyloxycarbonyl proline | Proline | 15 | 90 | 5 éq. $Et_2O/H_2O$ |
| 44 | NN' allyloxycarbonyl phénylparaméthoxy glycinate de terbutyle | N-phényl p méthoxy glycinate de terbutyle | 45 | 99 | 2,5 éq. |
| 45 | NN'allyloxycarbonyl méthylbenzylamine | N-méthyl, benzylamine | 5' | 23 % déprotection 77 %alkylation | 2,5 éq. $CH_3CN/H_2O$ |
| 46 | NN'allyloxycarbonyl méthylbenzylamine | N-méthyl, benzylamine | 5 | 75 % déprotection 25% alkylation | 6 éq. $CH_3CN/H_2O$ |

| Exemples | Substrats | Produits | Temps/ RT(mn) | $HNEt_2$ (éq) |
|---|---|---|---|---|
| 47 | 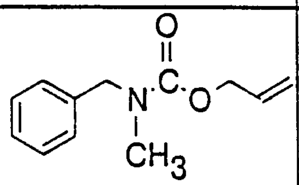 | 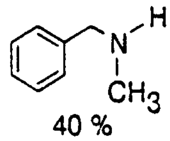 40 % 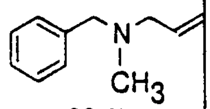 60 % | 5' | 2,2 pH : 3 |
| 48 | 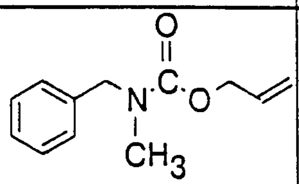 | 0 % 0 % | 5' à 0°C | 2,2 |
| 49 | 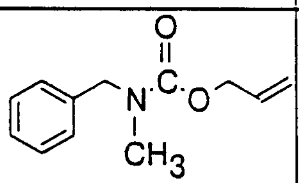 | 97 % 3 % | 5' | 40 |

Milieu : $Pd(OAc)_2$/TPPTS (1:2) ; $CH_3CN/H_2O$ (6:1)

| Exemples | Substrats | Produits | Temps/ RT(mn) | HNEt2 (éq) | Solvants |
|---|---|---|---|---|---|
| 50 | 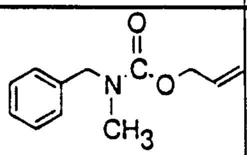 | 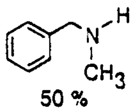 50 % 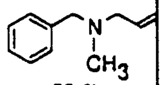 50 % | 5' | 2,2 | $Et_2O/H_2O$ |
| 51 | 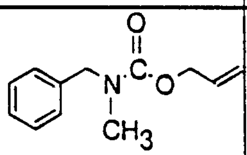 | 84 % 16 % | 5' | 5 | $Et_2O/H_2O$ |
| 52 | 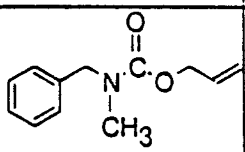 | 87 % 13 % | 5' | 8 | $Et_2O/H_2O$ |
| 53 | 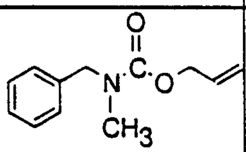 | 97 % 3 % | 5' | 40 | $Et_2O/H_2O$ |

Milieu : $Pd(OAc)_2$/TPPTS (1:2) ; $CH_3CN/H_2O$ (6:1)

| Exemples | Substrats | Produits | Temps/ RT(mn) | $HNEt_2$ (éq) | Solvants |
|---|---|---|---|---|---|
| 54 | 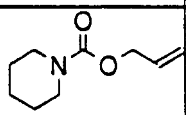 | 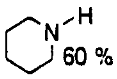 60 % 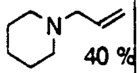 40 % | 5' | 2,2 | $CH_3CN$/ $H_2O$ |
| 55 | 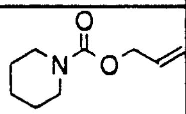 | 80 % 20 % | 5' | 6 | $CH_3CN$/ $H_2O$ |
| 56 | 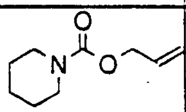 | 67 % 33 % | 5' | 2,5 | $CH_3CN$/ $H_2O$ |
| 57 | 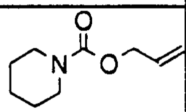 | 86 % 14 % | 5' | 6 | $CH_3CN$/ $H_2O$ |
| 58 | 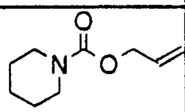 | 93 % 7 % | 5' | 15 | $CH_3CN$/ $H_2O$ |

Milieu : $Pd(OAc)_2$/TPPTS (1:2) ; $CH_3CN/H_2O$ (6:1)

| Exemples | Substrats | Produits | Temps/ RT | $HNEt_2^+$ (éq) | Rdt (%) |
|---|---|---|---|---|---|
| 59 | 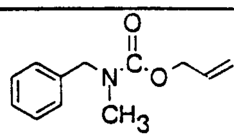 | 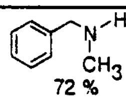 72 % 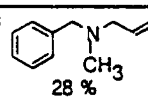 28 % | 15' | HCl 1,1 | 99 |
| 60 | 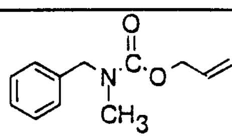 | 50 % 50 % | 15' | APTS 1,1 | 99 |
| 61 | 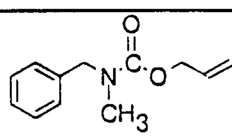 | 0 % 0 % | 18 h | AcOH 1,1 | traces |

Milieu : $Pd(OAc)_2$/TPPTS (1:2) 5 % ; $AcOEt/H_2O$ (6:1) + $HNEt_2$ (2,5 éq)..

| | | | | | |
|---|---|---|---|---|---|
| 62 | NN'allyloxycarbonyl méthylbenzyl amine | N-méthyl, benzylamine | 5' | 97 % déprotection 3 % alkylation | 40 éq. $CH_3CN/H_2O$ |
| 63 | N-allyloxycarbonyl pipéridine | pipéridine | 5' | 60 % déprotection 40 % alkylation | NuH 2,2 éq. $CH_3CN/H_2O$ |
| 64 | N-allyloxycarbonyl pipéridine | pipéridine | 2'30 | 80 % déprotection 20 % alkylation | 6 éq. $CH_3CN/H_2O$ |

**DEPROTECTION D'AMINES SECONDAIRES**

**Réactifs** : $Pd(OAC)_2$,TPPTS à température ambiante $Et_2O/H_2O$

| Exemples N° | Substrats | Produits obtenus | Temps en mn | Rendement | Rapport nucléophile/ substrat (solvant) |
|---|---|---|---|---|---|
| 65 | NN' allyloxycarbonyl phénylparaméthoxy glycinate de terbutyle | N-phényl p méthoxy glycinate de terbutyle | 45' | 100 % déprotection | 2,5 éq. ($Et_2O/H_2O$) |
| 66 | NN'allyloxycarbonyl méthylbenzylamine | N-méthyl benzylamine | 5' | 84 % déprotection 16 % alkylation | 5 éq.$Et_2O/H_2O$ |

| | | | | | |
|---|---|---|---|---|---|
| 67 | NN'allyloxycarbonyl méthylbenzylamine | N-méthyl benzylamine | 20' | 87 % déprotection 13 % alkylation | 8 éq.$Et_2O/H_2O$ |
| 68 | N-allyloxycarbonyl pipéridine | pipéridine | 10' | 86 % " " 14 % alkylation | 6 éq. $Et_2O/H_2O$ |
| 69 | N-allyloxycarbonyl pipéridine | pipéridine | 5' | 67 % déprotection 33 % alkylation | 2,5 éq.$Et_2O/H_2O$ |
| 70 | N-allyloxycarbonyl pipéridine | pipéridine | 10' | 93 % déprotection 7 % alkylation | 15 éq.$Et_2O/H_2O$ |
| 71 | NN'allyloxycarbonyl méthylbenzylamine | N-méthyl, benzylamine | 12' | 84 % " " 16 % alkylation | 5 eq. $Et_2O/H_2O$ |

| Exemples | Substrats | (éq) | Temps (min) Solvant | Produit | Rdt (%) |
|---|---|---|---|---|---|
| 72 | 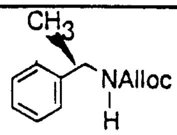 | $HNEt_2$ 5 | 15 THF | 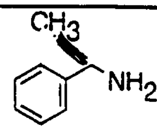 | 99 |
| 73 | 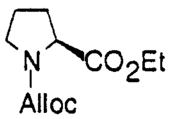 | $HNEt_2$ 5 | 60 THF | 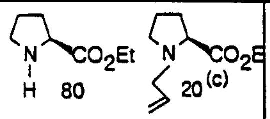 80 20(c) | 99 |
| 74 | 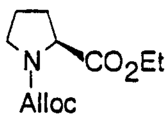 | $HNEt_2$ 15 | 20 THF | 97 3(c) | 99 |
| 75 | 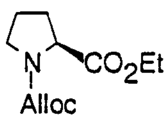 | $PhS^-Na^+$ 2 | 2 h EtOH | 100 0(c) | 99 |
| 76 | 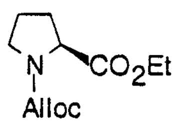 | 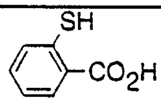 2 | 20 THF | 100 0(c) | 99 |
| 77 | 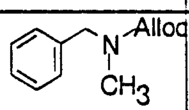 | 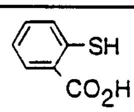 1.1 | 60 THF | 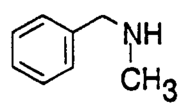 | 100 |
| 78 | 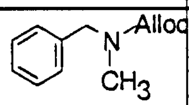 | 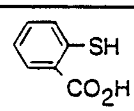 1.1 | 45 THF | 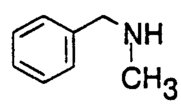 | 100 |
| 79 | 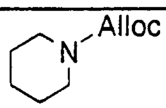 | 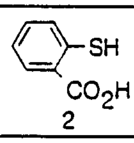 2 | 30 THF | 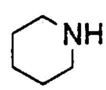 | 100 |
| 80 | 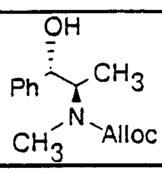 | 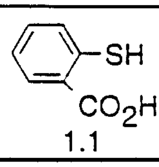 1.1 | 20 THF | 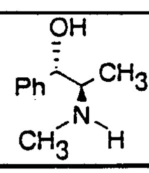 | 100 |

| Exemples | Substrats | Temps (min) | Produit | Rdt (%) |
|---|---|---|---|---|
| 81 | 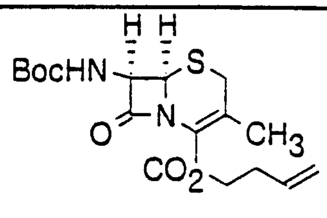 | 60 | 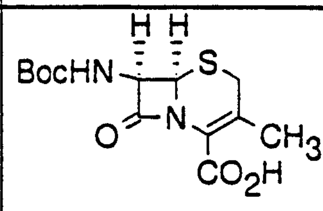 | 100 |
| 82 | 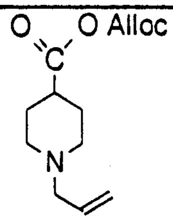 | 30 | 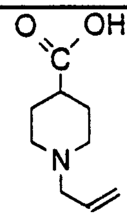 | 100 |
| 83 | 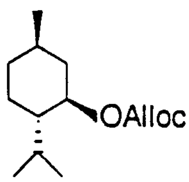 | 15 | 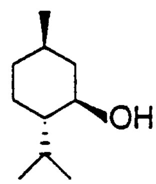 | 100 |
| 84 | 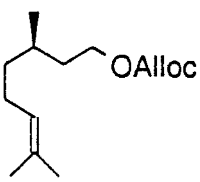 | 20 | 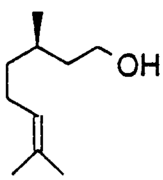 | 100 |
| 85 | 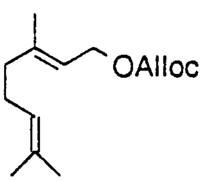 | 20 | 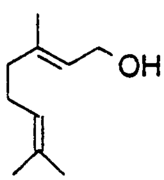 | 100 |

Déprotection de l'allyloxycarbonyl avec $Pd(dba)_2$, dppb, acide 2-thiobenzoïque

| Exemples | Substrats | Produit | Solvant | Temps | $HNEt_2$ (éq) | Rdt (%) |
|---|---|---|---|---|---|---|
| 86 | 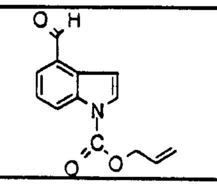 | 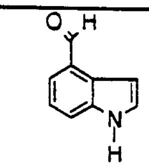 | $CH_3CN$ /$H_2O$ | 10' | 2,2 | 73 |
| 87 | 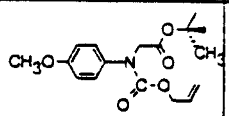 | 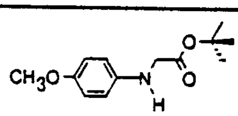 | $Et_2O$/ $H_2O$ | 45' | 2,5 | 99 |
| 88 | 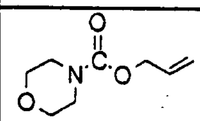 | 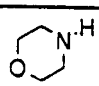 98 % 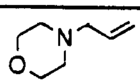 2 % | $CH_3CN$ /$H_2O$ | 5'<br>5' | 2,2<br>4,4 | 98<br>97 |
| 89 | 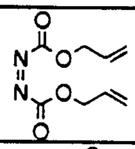 | NH=NH | $CH_3CN$ /$H_2O$ | 5' | 2,5 | 99 |
| 90 | 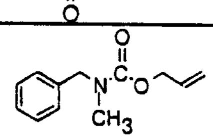 | 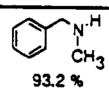 93.2 % 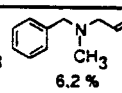 6.2 % | AcOEt/ $H_2O$ | 60' | 10 | 99 |
| 91 | 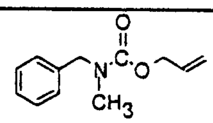 | 93,2 % 6,8 % | AcOEt/ $H_2O$ | 90' | 5 | 92* |
| 92 | 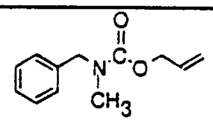 | 68 % 32 % | AcOEt/ $H_2O$ | 15' | 5 | 99 dégazé |
| 93 | 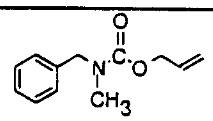 | 82 % 6 % | $C_3H_7C$ $N/H_2O$ | 15' | 10 | 88 |
| 94 | 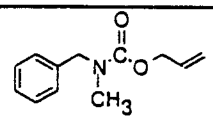 | 93 % 7 % | $C_3H_7C$ $N/H_2O$ | 15' | 5 | 99* |

Milieu : $Pd(OAc)_2$/TPPTS (1:2) 5 % ; $CH_3CN/H_2O$ (6:1)

* $Pd(OAc)_2$/TPPTS (1:2) 2,5 %

EXEMPLE 95 : LIBERATION DE LA PROLINE

| Substrat | Masse (g) | Nb de mole ($10^{-3}$M) | Solvant | Temps (RT) | $HNEt_2$ (éq) | % Pd |
|---|---|---|---|---|---|---|
| 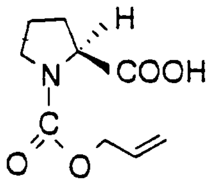 | 0,25 | 1,25 | $Et_2O/H_20$ | 15' | 2,2 | 3,26 |
| | 0,25 | 2,5 | $Et_2O/H_20$ | 15' | 2,2 | 1,63 |
| | 0,25 | 3,76 | $Et_2O/H_20$ | 15' | 2,2 | 1,088 |
| | 0,25 | 5,02 | $Et_2O/H_20$ | 30' | 2,2 | 0,88 |
| | 0,25 | 6,3 | $Et_2O/H_20$ | 60' | 2,2 | 0,65 |
| | 0,25 | 7,5 | $Et_2O/H_20$ | 60' | 2,2 | 0,55 |
| | 0,25 | 8,8 | $Et_2O/H_20$ | 90' | 2,2 | 0,46 |

Milieu : $Pd(OAc)_2$/TPPTS (1:2) ; $CH_3CN/H_2O$ (6:1).

Nous obtenons 0,9 grammes de L-proline (7,89 $10^{-3}$ M) soit un rendement de 90 % sur les 7 additions

EXEMPLE 98 : ESSAIS DIVERS

| Substrat | Nucléophile | Solvant* | Produits Obtenus | Durée | Tempé-rature | Rdt |
|---|---|---|---|---|---|---|
| 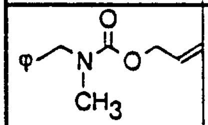 | $HNEt_2$ 5 éq | AcOEt/ $H_2O$ | 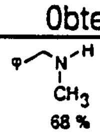 68 % 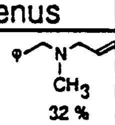 32 % | 15' | T.A | 100 % |
| 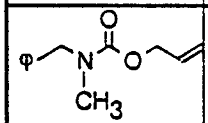 | $HNEt_2$ 10 éq | AcOEt/ $H_2O$ | 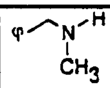 100 % 68 % | 30' | T.A | 100 % |
| 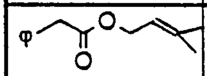 | $HNEt_2$ 5 éq | $CH_3CN$/ $H_2O$ | | 3 jours<br>4 jours | T.A<br>T.A | 75 %<br>90 % |
| 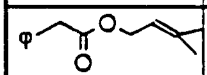 | $HNEt_2$ 5 éq | $CH_3CN$/ $H_2O$ | | 15' | 50°C | 100 % |
| 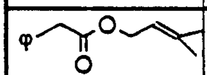 | $HNEt_2$ 5 éq | AcOEt/ $H_2O$ | Pas de déprotection | | T.A | <10 % |
| 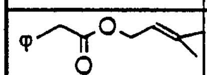 | $HNEt_2$ 5 éq | $CH_3CN$/ $H_2O$ | | 40' | 25°C | 100 % |
| 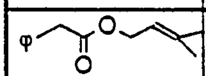 | $HNEt_2$ 5 éq | AcOEt/ $H_2O$ | Pratique-ment pas de déprotection | | 25°C | <10 % |

Pd(o) : Pd(AOc)$_2$/TPPTS (1:2) 5 % molaire

* solvant : dégazé

Conclusion : le nucléophile de choix pour les déprotections en milieu aqueux est $HNEt_2$. De plus, la diéthylamine est très volatile et s'élimine donc très facilement du milieu réactionnel. Lorsue le groupe protecteur est davantage encombré, il peut être nécessaire d'élever la température. Pour la déprotection d'acides carboxyliques le milieu homogène conduit à de bons résultats.

EXEMPLE 99 : APPLICATION A LA SYNTHESE PEPTIDIQUE

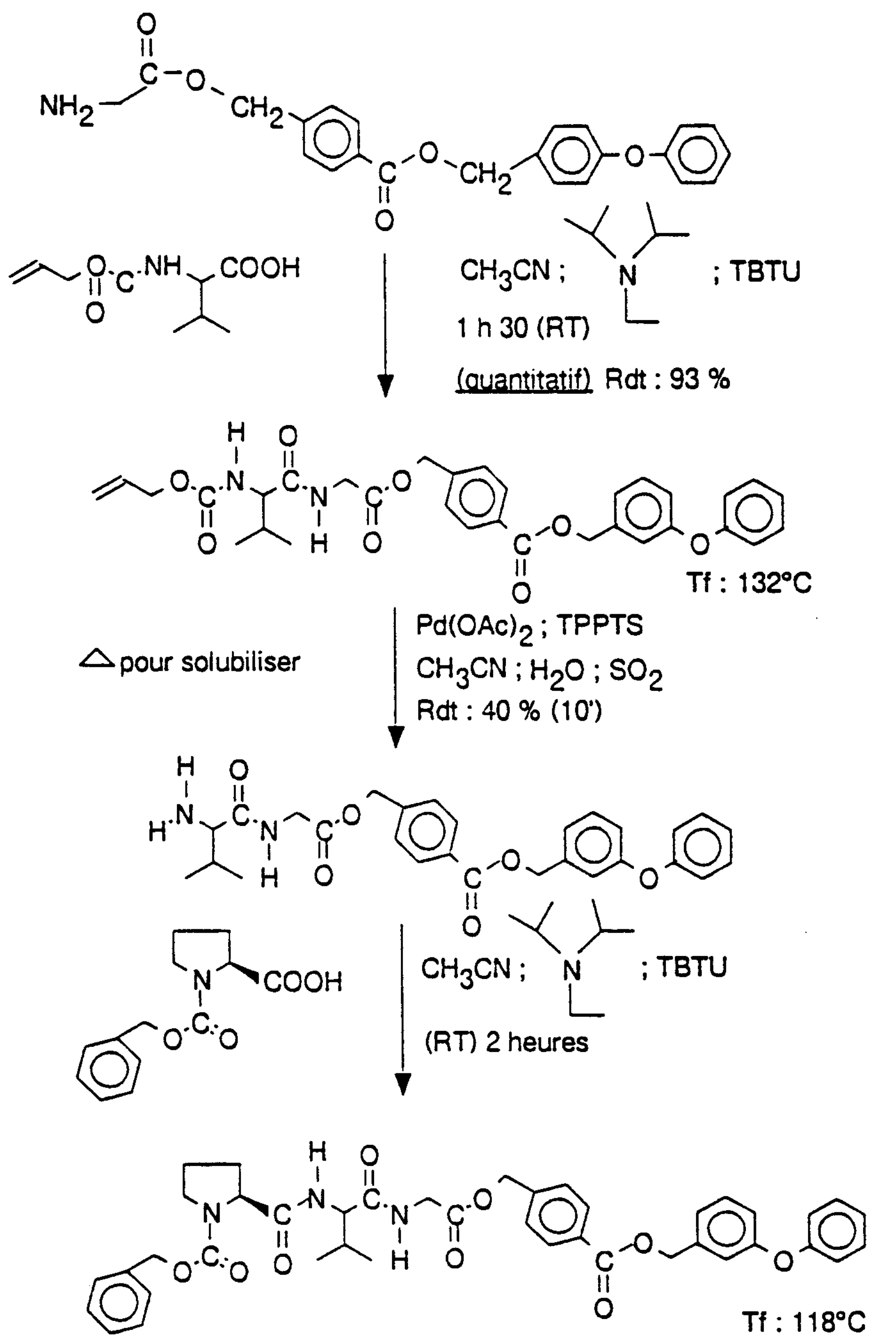


Produits : étudiés en 'H ; $C^{13}$ ; DEPTS ; Masse ; ...

| Substrat | Nucléophile | Solvant* | Produits Obtenus | Durée | Tempé-rature | Rdt |
|---|---|---|---|---|---|---|
| 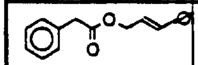 | $HNEt_2$ 5 éq | $CH_3CN/H_2O$ | 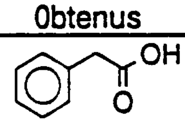 | 15' | T.A | 99 % |

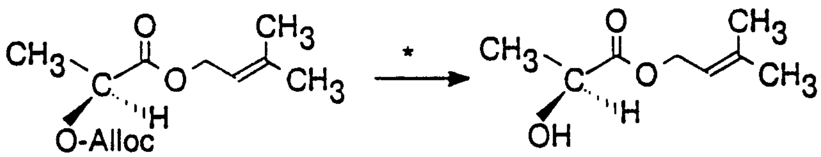


*

| Catalyseur | $Et_2NH$ | Solvant | Temps/ TA | Rdt |
|---|---|---|---|---|
| Pd(O) + DPPE : 1 % | 2,2 éq | EtOH | 6 h | 62 % |
| Pd(O) + TPPTS : 1 % | 2,2 éq | $CH_3CN/H_2O$ | 15' | 54 % |

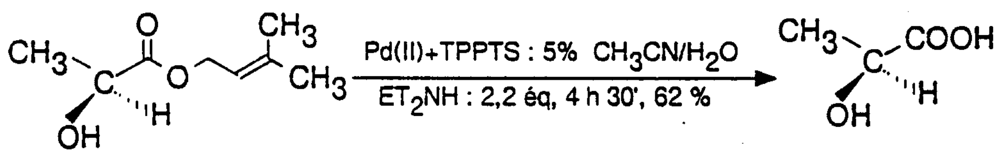


**Revendications**

1. Procédé de traitement de molécule comportant au moins une fonction alcoyloxycarbonyle insaturée en β et dont le reste, carbonyle inclus, comporte plus de 3 atomes de carbone, caractérisé par le fait que ladite molécule est soumise à un réactif qui comporte :

   a) phase aqueuse ou phase hydrophile ayant des propriétés de dissolution et de solvatation hydroïde ;
   b) un catalyseur comportant au moins un élément de la colonne VIII de la classification périodique des éléments, ledit élément de la colonne VIII de la classification périodique étant maintenu dans la phase aqueuse par complexation avec au moins un agent de coordination hydrosoluble ;
   c) un composé nucléophile hydrosoluble.

2. Procédé selon la revendication 1, caractérisé par le fait que ladite molécule présente une fonction nucléophile.

3. Procédé selon les revendications 1 et 2, caractérisé par le fait que ladite molécule présente un noyau aromatique.

4. Procédé selon les revendications 1 à 3, caractérisé par le fait que ladite molécule présente une fonction amine primaire ou secondaire.

5. Procédé selon les revendications 1 à 4, caractérisé par le fait que ladite molécule est chirale.

6. Procédé selon les revendications 1 à 5, caractérisé par le fait que ladite molécule est, ou présente comme chaînon, un motif choisi parmi les acides aminés, les peptides, les sucres et les (poly)nucléotides.

7. Procédé selon les revendications 1 à 6, caractérisé par le fait que ladite molécule présente un résidu tryptophane,

tyrosine, phénylalanine ou méthionine.

8. Procédé selon les revendications 1 à 7, caractérisé par le fait que ladite molécule présente au moins une fonction protégée par un groupe allyloxy carbonyle et au moins une fonction qui est protégée par un groupe distinct dudit allyloxycarbonyle.

9. Procédé selon les revendications 1 à 8, caractérisé par le fait que lesdits agents de coordination sont des dérivés trivalents hydrocarbonés des éléments de la colonne VB, avantageusement de période d'un rang supérieur à la deuxième.

10. Procédé selon les revendications 1 à 9, caractérisé par le fait que l'agent de coordination hydrosoluble est une pnictine, une trialcoylphosphine, de préférence une triarylphosphine greffée par des groupes hydrosolubilisant polaires.

11. Procédé selon les revendications 1 à 10, caractérisé par le fait que ledit composé nucléophile doit être tel que dans les conditions normales l'eau soit capable d'en dissoudre au moins 0,2 ; avantageusement 0,5 ; de préférence 1 équivalent de fonction nucléophile.

12. Procédé selon l'une des revendications 1 à 11, caractérisé par le fait que ledit composé nucléophile est présent (initialement mais plus préférablement en fin de réaction) à une concentration d'au moins 1/2, avantageusement 2, de préférence 5 équivalents par litre.

13. Procédé selon l'une des revendications 1 à 12, caractérisé par le fait que la quantité du dit nucléophile est au moins égale à 3/2 fois la quantité stoechiométriquement nécessaire.

14. Procédé selon l'une des revendications 1 à 13, caractérisé par le fait que la dite molécule comportant au moins une fonction alcoyloxycarbonyle répond à la formule suivante(III) :

$$Z\text{-}O\text{-}C(R_1)(R_2)\text{-}C(R_3){=}{=}C(R_4)(R_5) \qquad \text{(III)}$$

où

$R_1$ représente un hydrogène ou un radical alcoyle, de préférence à 1 ou 2 atomes de carbone ;
$R_2$ représente un hydrogène ou un radical alcoyle, de préférence à 1 ou 2 atomes de carbone ;
$R_3$ représente un hydrogène ou un radical alcoyle, de préférence à 1 ou 2 atomes de carbone, ou, avec $R_4$ forme une double liaison supplémentaire ;
$R_4$ représente un hydrogène ou un radical alcoyle, de préférence à 1 ou 2 atomes de carbone ou, avec $R_3$ forme une double liaison supplémentaire ;
$R_5$ représente un hydrogène ou un radical alcoyle, de préférence à 1 ou 2 atomes de carbone ;
Z un radical acyle y compris carbamyle, alcoxycarbonyle, thioalcoxycarbonyle.

15. Procédé selon la revendication 14, caractérisé par le fait que Z est de formule Z'-CO- avec Z' étant le radical issu de la molécule à protéger, la liaison remplaçant un hydrogène de la fonction dont on désire la protection.

16. Procédé selon la revendication 15, caractérisé par le fait que Z' est polyfonctionnel (en général au moins bi-, plus généralement au moins trifonctionnel).

17. Procédé selon la revendication 16, caractérisé par le fait que Z' est polyfonctionnel, les dites fonctions étant protégées par divers groupes protecteurs.

18. Procédé selon la revendication 17, caractérisé par le fait que Z' est de structure Z"-$\chi$- avec $\chi$ étant un atome autre que de carbone, avantageusement des colonnes V ou VI.

19. Procédé selon les revendications 1 à 18, caractérisé par le fait que ladite molécule présente une structure :

$$\zeta\ (\text{-CO-O-Allyl}_i)_n$$

avec i prenant toute les valeurs entières de 1 à n (n étant un nombre entier au moins égal à 2, de préférence à 3

et en général au plus égal à 100, voire à 50) -allyl$_i$ étant de formule

$$-C(R_1)(R_2)-C(R_3)==C(R_4)(R_5)$$

ζ étant le reste de la molécule polyfonctionelle.

**20.** Procédé selon la revendication 19, caractérisé par le fait que les allyl$_i$ sont tels qu'il y ait dans la molécule au moins deux, de préférence 3, formules différentes de allyl$_i$.

**21.** Procédé selon les revendications 1 à 20, caractérisé par le fait que la température réactionnelle est en général comprise entre le point de fusion finissante et le point d'ébullition commençante du milieu réactionnel, avantageusement entre 0°C et 100°C, de préférence entre l'ambiante et 50°C.

**22.** Procédé selon les revendications 1 à 21, caractérisé par le fait que ledit réactif présente est multiphasé.

**23.** Procédé selon les revendications 1 à 22, caractérisé par le fait que ledit réactif présente deux phases liquides, dont une phase aqueuse.

**24.** Procédé selon les revendications 1 à 23, caractérisé par le fait que ledit réactif comporte en outre un solvant A.

**25.** Procédé selon les revendications 1 à 24, caractérisé par le fait que ledit reste, carbonyle inclus, comporte plus de 5, de préférence plus de 10 atomes de carbone

**26.** Réactif utile pour cliver les fonctions alcoyloxycarbonyles insaturées :

a) une phase aqueuse ou phase hydrophile ayant des propriétés de dissolution et de solvatation hydroïde ;

b) un catalyseur comportant au moins un élément de la colonne VIII de la classification périodique des éléments, ledit élément de la colonne VIII de la classification périodique étant maintenu dans la phase aqueuse par complexation avec au moins un agent de coordination hydrosoluble ;

c) un composé nucléophile hydrosoluble.
caractérisé par le fait que ledit composé nucléophile est présent à une concentration d'au moins 1/2, avantageusement 2, de préférence 5 équivalents par litre et par le fait que ledit composé nucléophile hydrosoluble est choisi parmi ceux qui sur diverses échelles de nucléophilies en ont une supérieure à celle de l'ammoniac.

**27.** Réactif selon la revendication 26 :

a) une phase aqueuse ou phase hydrophile ayant des propriétés de dissolution et de solvatation hydroïde ;

b) un catalyseur comportant au moins un élément de la colonne VIII de la classification périodique des éléments, ledit élément de la colonne VIII de la classification périodique étant maintenu dans la phase aqueuse par complexation avec au moins un agent de coordination choisi parmi ;

- soit des phosphines dont la basicité est élevée, comme par exemples les bi- ou tri cyclohexylphosphines ;
- soit des pnictines polyfonctionnelles, en général bifonctionelle, permettant une chélation du métal par les fonctions pnictines ;

c) un composé nucléophile hydrosoluble dits "mou", dont la fonction nucléophile est à base de métalloïdes de rang élevé au moins égal à celui du phosphore ou du soufre et caractérisé par le fait que ledit composé nucléophile est présent à une concentration d'au moins 1/2, avantageusement 2, de préférence 5 équivalents par litre et par le fait que ledit composé nucléophile hydrosoluble est choisi parmi ceux qui sur diverses échelles de nucléophilies en ont une supérieure à celle de l'ammoniac.

**28.** Réactif selon la revendication 27, caractérisé par le fait que lesdites pnictines bifonctionelles sont telles que les fonctions pnictines sont, en prenant le chemin le plus direct, séparées par 2, 3 ou 4 atomes, le plus souvent de carbone ;

**29.** Réactif selon la revendication 28, caractérisé par le fait que lesdites pnictines bifonctionelles présente une formule du type ω, ω'diphenylphosphinoéthane; ou ω, ω'diphenyl-phosphinobutane.

**30.** Réactif selon les revendications 26 à 29, caractérisé par le fait que ledit composé nucléophile est rendu hydrosolubles en prévoyant une fonction fortement hydrophile sur la molécule.

**31.** Réactif selon la revendication 30, caractérisé par le fait que ledit composé nucléophile est rendu hydrosolubles en prévoyant comme fonction acide forte ou moyenne (pKa au plus égal à 6, avantageusement à 5, de préférence à 4).

**32.** Réactif selon la revendication 31, caractérisé par le fait que la fonction nucléophile est portée par un carbone postvicinal ou de préférence vicinal de celui portant la fonction acide.

**33.** Réactif selon les revendications 26 à 32, caractérisé par le fait que ledit composé nucléophile est l'acide thiosalicylique (H-S ΦCOOH), notamment sous forme acide ou monoanionique.

**34.** Réactif selon la revendication 26, caractérisé par le fait que ledit composé nucléophile est une amine secondaire, avantageusement la diéthylamine.

## Claims

**1.** Process for treating a molecule comprising at least one β-unsaturated alkoxycarbonyl functional group whose radical, including the carbonyl, Contains more than 3 carbon atoms, characterized in that the said molecule is subjected to the action of a reagent which comprises:

a) an aqueous phase or hydrophilic phase having water-like solvating and dissolving properties;
b) a catalyst comprising at least one element from group VIII of the Periodic Table of the Elements, the said element from group VIII of the Periodic Table being maintained in the aqueous phase by complexation with at least one water-soluble coordinating agent; and
c) a water-soluble nucleophilic compound.

**2.** Process according to claim 1, characterized in that the said molecule has a nucleophilic functional group.

**3.** Process according to claims 1 and 2, characterized in that the said molecule has an aromatic ring system.

**4.** Process according to claims 1 to 3, characterized in that the said molecule has a primary or secondary amine functional group.

**5.** Process according to claims 1 to 4, characterized in that the said molecule is chiral.

**6.** Process according to claims 1 to 5, characterized in that the said molecule is, or includes as chain member, a unit selected from amino acids, peptides, sugars and (poly)nucleotides.

**7.** Process according to claims 1 to 6, characterized in that the said molecule has a tryptophan, tyrosine, phenylalanine or methionine residue.

**8.** Process according to claims 1 to 7, characterized in that the said molecule has at least one functional group which is protected by an allyloxycarbonyl group and at least one functional group which is protected by a group other than the said allyloxycarbonyl.

**9.** Process according to claims 1 to 8, characterized in that the said coordinating agents are trivalent, hydrocarbon-containing derivatives of elements from group VB, advantageously in a period above the second row.

**10.** Process according to claims 1 to 9, characterized in that the water-soluble coordinating agent is a pnictine, a trialkylphosphine, or, preferably, a triarylphosphine grafted with polar water-solubilizing groups.

**11.** Process according to claims 1 to 10, characterized in that the said nucleophilic compound must be such that under standard conditions water is capable of dissolving it to the extent of at least 0.2, advantageously 0.5 and, preferably,

1 equivalent of the nucleophilic functional group.

**12.** Process according to one of claims 1 to 11, characterized in that the said nucleophilic compound is present (initially or, more preferably, at the end of the reaction) in a concentration of at least 1/2, advantageously 2 and, preferably, 5 equivalents per litre.

**13.** Process according to one of claims 1 to 12, characterized in that the quantity of the said nucleophile is equal to at least 3/2 times the required stoichiometric quantity.

**14.** Process according to one of claims 1 to 13, characterized in that the said molecule comprising at least one alkoxycarbonyl functional group corresponds to the following formula (III):

$$Z\text{-}O\text{-}C(R_1)(R_2)\text{-}C(R_3){=\!=}C(R_4)(R_5) \quad (III)$$

where

$R_1$ represents a hydrogen or an alkyl radical, preferably of 1 or 2 carbon atoms;
$R_2$ represents a hydrogen or an alkyl radical, preferably of 1 or 2 carbon atoms;
$R_3$ represents a hydrogen or an alkyl radical, preferably of 1 or 2 carbon atoms; or together with $R_4$ forms an additional double bond;
$R_4$ represents a hydrogen or an alkyl radical, preferably of 1 or 2 carbon atoms; or together with $R_3$ forms an additional double bond;
$R_5$ represents a hydrogen or an alkyl radical, preferably of 1 or 2 carbon atoms; and
Z an acyl radical, including carbamoyl, alkoxycarbonyl and thioalkoxycarbonyl.

**15.** Process according to claim 14, characterized in that Z is of formula Z'-CO- where Z' is the radical resulting from the molecule to be protected, the bond replacing a hydrogen of the functional group which it is desired to protect.

**16.** Process according to claim 15, characterized in that Z' is polyfunctional (generally at least difunctional, more generally at least trifunctional).

**17.** Process according to claim 16, characterized in that Z' is polyfunctional, the said functional groups being protected by various protecting groups.

**18.** Process according to claim 17, characterized in that Z' is of structure Z"-$\chi$- in which $\chi$ is an atom other than carbon, advantageously from group V or VI.

**19.** Process according to claims 1 to 18, characterized in that the said molecule has a structure:

$$\zeta(\text{-CO-O-allyl}_i)_n$$

in which i is any integer from 1 to n (n being an integer of at least 2, preferably 3 and, in general, of not more than 100, or even not more than 50); -allyl$_i$ is of formula

$$\text{-}C(R_1)(R_2)\text{-}C(R_3){=\!=}C(R_4)(R_5)$$

; and $\zeta$ is the radical of the polyfunctional molecule.

**20.** Process according to claim 19, characterized in that the allyl$_i$s are such that within the molecule there are at least two, preferably 3, different formulae of allyl$_i$.

**21.** Process according to claims 1 to 20, characterized in that the reaction temperature is generally between the final melting point and the starting boiling point of the reaction medium, advantageously between 0°C and 100°C and, preferably, between ambient and 50°C.

**22.** Process according to claims 1 to 21, characterized in that the said reagent is multiphase.

**23.** Process according to claims 1 to 22, characterized in that the said reagent has two liquid phases of which one

phase is aqueous.

**24.** Process according to claims 1 to 23, characterized in that the said reagent additionally comprises a solvent A.

**25.** Process according to claims 1 to 24, characterized in that the said radical, including the carbonyl, contains more than 5 and, preferably, more than 10 carbon atoms.

**26.** Reagent useful for cleaving unsaturated alkoxycarbonyl functional groups, which comprises:

a) an aqueous phase or hydrophilic phase having water-like solvating and dissolving properties;
b) a catalyst comprising at least one element from group VIII of the Periodic Table of the Elements, the said element from group VIII of the Periodic Table being maintained in the aqueous phase by complexation with at least one water-soluble coordinating agent; and
c) a water-soluble nucleophilic compound,
characterized in that the said nucleophilic compound is present in a concentration of at least 1/2, advantageously 2 and, preferably, 5 equivalents per litre and in that the said water-soluble nucleophilic compound is selected from those which on various scales of nucleophilicity have a nucleophilicity greater than that of ammonia.

**27.** Reagent according to claim 26, which comprises:

a) an aqueous phase or hydrophilic phase having water-like solvating and dissolving properties;
b) a catalyst comprising at least one element from group VIII of the Periodic Table of the Elements, the said element from group VIII of the Periodic Table being maintained in the aqueous phase by complexation with at least one coordinating agent selected from;

- either phosphines of high basicity such as, for example, di- or tricyclohexylphosphines;
- or polyfunctional pnictines, generally difunctional, which allow chelation of the metal by the pnictine functional groups;

c) a water-soluble nucleophilic compound, which is termed "soft", whose nucleophilic functional group is based on metalloids of a high rank which is at least equal to that of phosphorus or of sulphur, and characterized in that the said nucleophilic compound is present in a concentration of at least 1/2, advantageously 2 and, preferably, 5 equivalents per litre and in that the said water-soluble nucleophilic compound is selected from those which on various scales of nucleophilicity have a nucleophilicity which is greater than that of ammonia.

**28.** Reagent according to claim 27, characterized in that the said difunctional pnictines are such that the pnictine functional groups are separated - taking the most direct route - by 2, 3 or 4 atoms, usually of carbon.

**29.** Reagent according to claim 28, characterized in that the said difunctional pnictines have a formula of the $\omega,\omega'$-diphenylphosphinoethane or $\omega,\omega'$-diphenylphosphinobutane type.

**30.** Reagent according to claims 26 to 29, characterized in that the said nucleophilic compound is rendered water-soluble by providing on the molecule a strongly hydrophilic functional group.

**31.** Reagent according to claim 30, characterized in that the said nucleophilic compound is rendered water-soluble by providing a strongly or moderately acidic functional group (pKa of not more than 6, advantageously not more than 5 and, preferably, not more than 4).

**32.** Reagent according to claim 31, characterized in that the nucleophilic functional group is carried by a carbon which is post-vicinal or, preferably, vicinal to that carrying the acidic functional group.

**33.** Reagent according to claims 26 to 32, characterized in that the said nucleophilic compound is thiosalicylic acid (H-S-$\Phi$COOH), especially in acid form or monoanionic form.

**34.** Reagent according to claim 26, characterized in that the said nucleophilic compound is a secondary amine, advantageously diethylamine.

## Patentansprüche

1. Verfahren zur Behandlung eines Moleküls, welches mindestens eine β-ungesättigte Alkyloxycarbonylfunktion umfaßt und wovon der Rest einschließlich Carbonyl mehr als 3 Kohlenstoffatome umfaßt, dadurch gekennzeichnet, daß dieses Molekül einem Reagens ausgesetzt wird, das umfaßt:

   a) wäßrige Phase oder hydrophile Phase mit Lösungs- und wasserähnlichen Solvatationseigenschaften,
   b) einen Katalysator, der mindestens ein Element der Gruppe VIII des Periodensystems der Elemente umfaßt, wobei das Element der Gruppe VIII des Periodensystems in der wäßrigen Phase durch Komplexierung mit mindestens einem wasserlöslichen Koordinationsmittel gehalten wird,
   c) eine wasserlösliche nukleophile Verbindung.

2. Verfahren nach Anspruch 1, dadurch gekennzeichnet, daß das Molekül eine nukleophile Funktion aufweist.

3. Verfahren nach den Ansprüchen 1 und 2, dadurch gekennzeichnet, daß das Molekül einen aromatischen Kern aufweist.

4. Verfahren nach den Ansprüchen 1 bis 3, dadurch gekennzeichnet, daß das Molekül eine primäre oder sekundäre Aminfunktion aufweist.

5. Verfahren nach den Ansprüchen 1 bis 4, dadurch gekennzeichnet, daß das Molekül chiral ist.

6. Verfahren nach den Ansprüchen 1 bis 5, dadurch gekennzeichnet, daß das Molekül ein Grundbaustein, ausgewählt aus den Aminosäuren, Peptiden, Zuckern und (Poly)nukleotiden, ist oder einen solchen Grundbaustein als Kettenglied aufweist.

7. Verfahren nach den Ansprüchen 1 bis 6, dadurch gekennzeichnet, daß das Molekül einen Tryptophan-, Tyrosin-, Phenylalanin- oder Methioninrest aufweist.

8. Verfahren nach den Ansprüchen 1 bis 7, dadurch gekennzeichnet, daß das Molekül mindestens eine durch eine Allyloxycarbonylgruppe geschützte Funktion und mindestens eine Funktion, die durch eine Gruppe, die von der Allyloxycarbonylgruppe verschieden ist, geschützt ist, aufweist.

9. Verfahren nach den Ansprüchen 1 bis 8, dadurch gekennzeichnet, daß die Koordinationsmittel dreiwertige kohlenwasserstoffhaltige Derivate der Elemente der Gruppe VB, vorteilhafterweise aus einer Periode eines höheren Rangs als der zweiten sind.

10. Verfahren nach den Ansprüchen 1 bis 9, dadurch gekennzeichnet, daß das wasserlösliche Koordinationsmittel ein Pnictin, ein Trialkylphosphin, vorzugsweise ein durch Wasserlöslichkeit vermittelnde polare Gruppen gepfropftes Triarylphosphin ist.

11. Verfahren nach den Ansprüchen 1 bis 10, dadurch gekennzeichnet, daß die nukleophile Verbindung so sein muß, daß unter normalen Bedingungen Wasser in der Lage ist, mindestens 0,2, vorteilhafterweise 0,5, vorzugsweise 1 Äquivalent nukleophiler Funktionen zu lösen.

12. Verfahren nach einem der Ansprüche 1 bis 11, dadurch gekennzeichnet, daß die nukleophile Verbindung (anfänglich, aber mehr bevorzugt am Ende der Reaktion) in einer Konzentration von mindestens 1/2, vorteilhafterweise 2, vorzugsweise 5 Äquivalenten pro Liter vorliegt.

13. Verfahren nach einem der Ansprüche 1 bis 12, dadurch gekennzeichnet, daß die Menge des Nukleophils mindestens die eineinhalbfache der erforderlichen stöchiometrischen Menge ist.

14. Verfahren nach einem der Ansprüche 1 bis 13, dadurch gekennzeichnet, daß das Molekül, das mindestens eine Alkyloxycarbonylfunktion umfaßt, der folgenden Formel (III) entspricht:

$$Z\text{-}O\text{-}C(R_1)(R_2)\text{-}C(R_3){=\!=}C(R_4)(R_5) \qquad \text{(III)},$$

   in der

$R_1$ für ein Wasserstoffatom oder einen Alkylrest, vorzugsweise mit 1 oder 2 Kohlenstoffatomen, steht,
$R_2$ für ein Wasserstoffatom oder einen Alkylrest, vorzugsweise mit 1 oder 2 Kohlenstoffatomen, steht,
$R_3$ für ein Wasserstoffatom oder einen Alkylrest, vorzugsweise mit 1 oder 2 Kohlenstoffatomen, steht oder mit $R_4$ eine zusätzliche Doppelbindung bildet,
$R_4$ für ein Wasserstoffatom oder einen Alkylrest, vorzugsweise mit 1 oder 2 Kohlenstoffatomen, steht oder mit $R_3$ eine zusätzliche Doppelbindung bildet,
$R_5$ für ein Wasserstoffatom oder einen Alkylrest, vorzugsweise mit 1 oder 2 Kohlenstoffatomen, steht,
Z ein Acylrest, umfassend Carbamyl, Alkoxycarbonyl, Thioalkoxycarbonyl, ist.

**15.** Verfahren nach Anspruch 14, dadurch gekennzeichnet, daß Z die Formel Z'-CO- aufweist, wobei Z' der Rest ist, der aus dem zu schützenden Molekül stammt, wobei die Bindung ein Wasserstoffatom der Funktion, die man zu schützen wünscht, ersetzt.

**16.** Verfahren nach Anspruch 15, dadurch gekennzeichnet, daß Z' polyfunktionell (im allgemeinen zumindest di-, allgemeiner mindestens trifunktionell) ist.

**17.** Verfahren nach Anspruch 16, dadurch gekennzeichnet, daß Z' polyfunktionell ist, wobei diese Funktionen durch unterschiedliche Schutzgruppen geschützt sind.

**18.** Verfahren nach Anspruch 17, dadurch gekennzeichnet, daß Z' die Struktur Z"-$\chi$- aufweist, wobei $\chi$ ein anderes Atom als Kohlenstoff, vorzugsweise aus den Gruppen V oder VI, ist.

**19.** Verfahren nach den Ansprüchen 1 bis 18, dadurch gekennzeichnet, daß das Molekül eine Struktur:

$$\zeta(\text{-CO-O-allyl}_i)_n$$

aufweist, wobei i alle ganzen Zahlenwerte von 1 bis n annimmt (wobei n eine ganze Zahl von mindestens gleich 2, vorzugsweise mindestens gleich 3 und im allgemeinen von höchstens gleich 100, beispielsweise 50 ist),
-allyl$_i$ die Formel

$$\text{-C}(R_1)(R_2)\text{-C}(R_3){=\!=}\text{C}(R_4)(R_5)$$

aufweist, wobei $\zeta$ der Rest des polyfunktionellen Moleküls ist.

**20.** Verfahren nach Anspruch 19, dadurch gekennzeichnet, daß die Reste allyl$_i$ dergestalt sind, daß es in dem Molekül mindestens zwei vorzugsweise 3 unterschiedliche Formeln für allyl$_i$ gibt.

**21.** Verfahren nach den Ansprüchen 1 bis 20, dadurch gekennzeichnet, daß die Reaktionstemperatur im allgemeinen zwischen dem abschließenden Schmelzpunkt und dem anfänglichen Siedepunkt des Reaktionsmediums, vorteilhafterweise zwischen 0°C und 100°C, vorzugsweise zwischen Umgebungstemperatur und 50°C liegt.

**22.** Verfahren nach den Ansprüchen 1 bis 21, dadurch gekennzeichnet, daß das vorliegende Reagens in mehreren Phasen vorliegt.

**23.** Verfahren nach den Ansprüchen 1 bis 22, dadurch gekennzeichnet, daß das Reagens zwei flüssige Phasen aufweist, von denen eine Phase wäßrig ist.

**24.** Verfahren nach den Ansprüchen 1 bis 23, dadurch gekennzeichnet, daß das Reagens darüber hinaus ein Lösemittel A umfaßt.

**25.** Verfahren nach den Ansprüchen 1 bis 24, dadurch gekennzeichnet, daß der Rest einschließlich Carbonyl mehr als 5, vorzugsweise mehr als 10 Kohlenstoffatome umfaßt.

**26.** Reagens, das zum Abspalten der ungesättigten Alkyloxycarbonylfunktionen geeignet ist:

a) eine wäßrige Phase oder hydrophile Phase mit Lösungs- und wasserähnlichen Solvatationseigenschaften,
b) ein Katalysator, der mindestens ein Element der Gruppe VIII des Periodensystems der Elemente umfaßt, wobei das Element der Gruppe VIII des Periodensystems in der wäßrigen Phase durch Komplexierung mit

mindestens einem wasserlöslichen Koordinationsmittel gehalten wird,

c) eine wasserlösliche nukleophile Verbindung, dadurch gekennzeichnet, daß die nukleophile Verbindung in einer Konzentration von mindestens 1/2, vorteilhafterweise 2, vorzugsweise 5 Äquivalenten pro Liter vorliegt, und dadurch, daß die wasserlösliche nukleophile Verbindung ausgewählt ist unter jenen, die bei unterschiedlichen Nukleophiliegraden einen höheren als Ammoniak aufweisen.

**27.** Reagens nach Anspruch 26:

a) eine wäßrige Phase oder hydrophile Phase mit Lösungs- und wasserähnlichen Solvatationseigenschaften,
b) ein Katalysator, der mindestens ein Element der Gruppe VIII des Periodensystems der Elemente umfaßt, wobei das Element der Gruppe VIII des Periodensystems in der wäßrigen Phase gehalten wird durch Komplexierung mit mindestens einem Koordinationsmittel, ausgewählt unter:

- entweder den Phosphinen, deren Basizität erhöht ist, wie beispielsweise den Di- oder Tricyclohexylphosphinen,
- oder den polyfunktionellen, im allgemeinen difunktionellen Pnictinen, die eine Chelatbildung mit dem Metall durch die Pnictinfunktionen ermöglichen,

c) eine als "schwach" bezeichnete wasserlösliche nukleophile Verbindung, deren nukleophile Funktion auf Nichtmetallen von höherem Rang mindestens gleich jenem von Phosphor oder Schwefel beruht,
und dadurch gekennzeichnet, daß die nukleophile Verbindung in einer Konzentration von mindestens 1/2, vorteilhafterweise 2, vorzugsweise 5 Äquivalenten pro Liter vorliegt, und dadurch, daß die wasserlösliche nukleophile Verbindung ausgewählt ist unter jenen, die bei unterschiedlichen Nukleophiliegraden einen höheren als Ammoniak aufweisen.

**28.** Reagens nach Anspruch 27, dadurch gekennzeichnet, daß die difunktionellen Pnictine dergestalt sind, daß die Pnictinfunktionen, wenn man den direktesten Weg nimmt, durch 2, 3 oder 4 Atome, überaus häufig Kohlenstoffatome, voneinander getrennt sind.

**29.** Reagens nach Anspruch 28, dadurch gekennzeichnet, daß die difunktionellen Pnictine eine Formel des Typs $\omega,\omega'$-Diphenylphosphinoethan oder $\omega,\omega'$-Diphenylphosphinobutan aufweisen.

**30.** Reagens nach den Ansprüchen 26 bis 29, dadurch gekennzeichnet, daß die nukleophile Verbindung wasserlöslich gemacht wird, indem eine stark hydrophile Funktion an dem Molekül vorgesehen wird.

**31.** Reagens nach Anspruch 30, dadurch gekennzeichnet, daß die nukleophile Verbindung wasserlöslich gemacht wird, indem als Funktion eine starke oder mittelstarke Säurefunktion (pKa höchstens gleich 6, vorteilhafterweise höchstens gleich 5, vorzugsweise höchstens gleich 4) vorgesehen wird.

**32.** Reagens nach Anspruch 31, dadurch gekennzeichnet, daß die nukleophile Funktion von einem Kohlenstoffatom getragen wird, das postvicinal oder vorzugsweise vicinal zu jenem, das die Säurefunktion trägt, ist.

**33.** Reagens nach den Ansprüchen 26 bis 32, dadurch gekennzeichnet, daß die nukleophile Verbindung Thiosalicylsäure (H-S ΦCOOH), insbesondere in Säure- oder Monoanionform, ist.

**34.** Reagens nach Anspruch 26, dadurch gekennzeichnet, daß die nukleophile Verbindung ein sekundäres Amin, vorteilhafterweise Diethylamin ist.